# EUROPEAN PATENT SPECIFICATION

(11) **EP 4 157 099 B1**
(45) Date of publication and mention of the grant of the patent: **05.11.2025**
(21) Application number: 21729315.8
(22) Date of filing: 28.05.2021
(51) Int. Cl.: A61B 17/00, A61F 2/24

(54) **DEVICE FOR HEART REPAIR**
VORRICHTUNG ZUR HERZREPARATUR
DISPOSITIF DE RÉPARATION CARDIAQUE

(30) Priority: 02.06.2020 GB 202008286
(43) Date of publication of application: 05.04.2023
(73) Proprietor: Cardiomech AS, 7030 Trondheim (NO)
(72) Inventor: HIORTH, Nikolai, 0779 Oslo (NO); HIORTH, Hans Emil, 7030 Trondheim (NO)
(74) Representative: Dehns
(86) International application number: PCT/EP2021/064443
(87) International publication number: WO 2021/244987

(56) References cited:
- EP-A1- 1 929 960
- EP-A1- 1 929 960
- WO-A1-01/08717
- WO-A1-01/08717
- WO-A1-2012/006375
- WO-A1-2018/089838
- JP-B2- 5 145 219
- JP-B2- 5 145 219
- US-A1- 2004 078 054
- US-A1- 2004 078 054
- US-A1- 2005 234 512
- US-A1- 2005 234 512
- US-A1- 2019 343 634
- US-A1- 2019 343 634
- US-B2- 10 660 633
- US-B2- 10 660 633

## Description

The present invention relates to an anchor configuration for implantation in body tissue, such as in context of a device for implanting an artificial chordae line in order to repair a heart valve, as well as to related methods.

The chordae tendineae are cord-like tendons that connect the papillary muscles to the tricuspid valve and the mitral valve in the heart. The valves consist of leaflets that open and close with the beating of the heart in order to control blood flow and blood pressure within the heart.

Mitral valve disease presents an important challenge to cardiac surgeons and cardiologists. Mitral regurgitation has become the leading pathophysiological condition of the mitral valve in the developed world. One of the most important causes of regurgitation is prolapse of one of the mitral leaflets. The pathological abnormality that requires repair is rupture or other degenerative changes of the chords, leaflet or other related structures. When the chord(s) remain intact, the mitral leaflets open and close synchronously and in a fashion that prevents leakage of the valve. The normal chords can rupture acutely causing acute decompensation, in the form of, heart failure. This usually results in an emergency condition requiring rapid intervention. Damage to the chord(s) can also occur more slowly including rupturing or elongation due to degenerative processes, causing the mitral valve to develop leaks or regurgitation.

Surgical repair of the mitral valve has become relatively standardized, using resection of the prolapsed leaflet and/or implantation of new, artificial chordae lines to control leaflet motion. In addition a mitral ring is frequently placed to shrink the size of the mitral valve annulus. Surgical replacement of ruptured or elongated chords is highly effective in eliminating or minimizing mitral valve regurgitation. The procedure is presently performed with open heart surgery techniques. This requires use of cardiopulmonary bypass and arresting of the heart. This surgical approach, although working well, is a highly invasive procedure which can cause serious complications, long hospital stays and substantial expense. Consequently a less invasive approach would be preferable.

Insertion of mitral leaflet chords has been done using a minimally invasive surgical approach entering the heart through its apex. The technique, was developed by the company Neochord Inc. and is described, for example, in WO 2012/167120, but still requires a surgical incision and the chords do not get inserted in the papillary muscles where they normally should be fixed.

WO 2008/101113 describes another example of a system for repair of the heart, including implantation of artificial chordae lines. In the described method an anchor can be attached to the papillary muscle and is coupled to the leaflet of the mitral valve by an artificial chordae line, a suture and a clip. The clip allows for adjustment of the length of the artificial chordae line. A complex multi-stage process is required to implant the papillary anchor and the suture and join them together. The papillary anchor is formed of a memory metal such as nitinol and has a 'flowered' shape with sharp 'petals' for hooking the anchor to body tissue. The flowered shape is flattened into a tube shape and held in a tube that is passed into the heart. The tube and anchor are then pressed against the papillary muscle and the anchor is pushed out of the tube so that the petals pierce the muscle and fold outward through the muscle to provide a secure coupling of the anchor to the muscle tissue. In a subsequent surgical procedure, an artificial chordae line may be attached to the anchor. Then in a further step, the suture is attached to the leaflet and this suture is joined to the chord by the clip. The suture is attached to the leaflet by locating a vacuum port near to the leaflet and pulling it into the vacuum port where it can be pierced.

It will be appreciated that this technique, whilst avoiding open heart surgery, still requires a sequence of relatively complex steps. The number of steps required increases the risk. Furthermore, the complexity of the device means that parts implanted within the body are at risk of coming loose and injuring the patient by embolization. In particular, the clip could come loose from the anchors. It is also thought that the use of a suture with an additional clip, as proposed, may not effectively repair the heart valve since it will not closely simulate a natural chord.

In an earlier patent application, WO2016/042022, the present applicant disclosed a catheter device for implanting an artificial chordae line to repair a heart valve. The catheter device of WO2016/042022 includes a mechanical gripper device for grasping the leaflet of the heart valve, with a leaflet anchor housed in the gripper. The leaflet anchor can be formed from a flexible material, such as nitinol, with a grapple hook shape in an unfolded configuration, and being able to deform elastically into the folded configuration, for example when constrained within a leaflet anchor channel in the gripper device. The hooks are straightened out when the leaflet anchor is in the folded configuration. When the leaflet is grasped by the gripper device then the leaflet anchor can be pushed out of the gripper to drive the hooks though the leaflet whilst they return elastically to the unfolded configuration, thereby securing the leaflet anchor in the leaflet.

The device described in WO2016/042022 also uses a papillary anchor with a broadly similar arrangement of foldable hooks. The papillary anchor is held within a tube of the catheter device in a folded configuration and can be pushed out of the tube with the hooks being driven through the papillary muscle whilst they return elastically to the unfolded configuration, thereby securing the papillary anchor to the muscle. The papillary anchor includes a locking ring acting as a locking mechanism for clamping an artificial chordae line when no force is applied. The locking ring maybe elastically deformed to release the line from the locking mechanism for adjustment of the length of the chordae line.

Whilst the device of WO2016/042022 provided a significant advance in this field it has been found that further refinement of the design may be advantageous. The present disclosure relates to new features building on the design of the device disclosed in WO2016/042022 in various respects.

JP 5145219 B2 discloses a surgical fastener in the form of a surgical staple for attaching a graft or a mesh to body tissue.

US 10660633 B2 discloses a suture anchor for attaching soft tissue to bone.

WO 01/08717 A1 discloses an implantable device for delivering a drug to a target location.

US 2005/234512 A1 discloses an endoscopic fastening assembly comprising anchors for deployment in target tissue.

US 2019/343634 A1 discloses a tissue anchor for heart valve repair comprising a plurality of radially extending arm portions.

EP 1929960 A1 discloses a suture clip for use in combination with an endoscope.

US 2004/078054 A1 discloses a plurality of anchors in combination with a cord for reducing a lung volume.

In accordance with the present invention an anchor configuration as discussed in the first aspect, an anchor in combination with a line as discussed in the second aspect, and a method of manufacture of an anchor in combination with a line as discussed in the thirteenth aspect, are herein provided.

Viewed from a first aspect the invention provides an anchor configuration as claimed in claim 1. The anchor configuration comprises: an anchor comprising a number of hooks for engagement with the body tissue and having a folded and unfolded position, wherein the anchor is made of an elastic material such that it can be elastically deformed into the folded position by application of a constraining force, and will return to the unfolded position when no constraining force is applied; and a plugging device for enhancing contact with the body tissue; wherein the plugging device is for combining with one or more parts of the anchor to provide the enhanced contact; wherein at least one of the hooks is encircled by the plugging device; wherein the plugging device is a line, the line in combination with the anchor; wherein the line is joined to the anchor by a knotting configuration comprising a plurality of loops around the anchor; wherein the at least one hook is encircled by at least one loop of the plurality of loops; and wherein the line is configured to seal an entry site between the hooks and the body tissue when the anchor is implanted in the body tissue.

Upon implantation of a conventional anchor in body tissue, the hooks engage the body tissue via entry sites. In situations where the hooks completely pass through the tissue during engagement, a narrow channel and/or passageway may result around each hook. Where the tissue is adjacent to a fluid of a sufficient fluid pressure, for example blood during contraction of the heart in the cardiac cycle, the fluid may be forced through the entry sites, such as the narrow channel and/or passageway, and produce a high velocity jet. For example, if the anchor was implanted in a mitral valve of a heart, the circulation of blood through the mitral valve may be of sufficient pressure such that a high velocity jet of blood is forced through each location where a hook of the anchor engages the mitral valve. A flow of blood to each location where a hook of the anchor engages the mitral valve to produce the high velocity jets may pass between the anchor and the body tissue and/or may drive the anchor away from the body tissue it is implanted in. This high velocity jet and/or a flow of blood producing the high velocity jet may impede tissue growth at the site of implantation in body tissue and thus weaken the resultant tensile strength of the implanted anchor. Additionally, any seepage or leakage of a fluid through such entry sites may increase the time which it takes for the body tissue to heal.

By providing a plugging device that combines with part(s) of the anchor to enhance contact with the body tissue, the plugging device may close openings through the body tissue at entry sites produced by the hooks during engagement with body tissue. The plugging device may act to increase a cross-sectional area of the anchor configuration, for example by being placed around one or more part(s) of the anchor, such that flow through the entry sites is impeded. At least impeding the flow may reduce movement of the implanted anchor due to flow through the entry sites. Impeding or preventing the flow of fluid through the hook entry sites may facilitate tissue growth around the site where the anchor is implanted in body tissue and increase the overall tensile strength of the anchor when implanted in body tissue. In an example where the anchor is implanted in a leaflet of a mitral valve, if a main body of the anchor and the plugging device is on the high pressure side then the blood pressure can push the anchor and/or the plugging device toward the entry sites of the hooks, hence forming a strong barrier with a sealing effect preventing unwanted blood flow through the entry sites.

Requiring that at least one of the hooks is encircled by the plugging device may provide localised sealing, or impeding of flow, to the hooks which are encircled by the plugging device. This may help stabilise these hooks, and reduce motion of the anchor overall when implanted in the body tissue. As the openings at the entry sites originate due to the engagement of the hooks with the body tissue, encircling at least one of the hooks using the plugging device may aid sealing of the entry sites of the at least one hook, as well as also aiding tissue re-growth and/or aiding sealing of adjacent entry sites. The localised sealing and/or impeding effect provided by the plugging device around each of these hooks against flow through the entry sites may be in addition to the general impeding of flow provided by the plugging device.

The plugging device may encircle at least two of the hooks. The plugging device may encircle each of the number of hooks. For example, an anchor may have only two hooks, or some other number of hooks with each hook encircled by the plugging device. The use of the plugging device encircling each hook can give a similar treatment for each entry site of the hooks.

Additionally, the plugging device may be configured to facilitate tissue ingrowth around the anchor, such that the anchor is better secured to the body tissue it is implanted in. The plugging device may provide additional surface area for tissue to grow around. The plugging device may additionally be of a biocompatible material which encourages tissue ingrowth. It will be appreciated that the plugging device may encourage tissue ingrowth regardless of whether the anchor is subject to high velocity jets and/or fluid flows resulting from engagement of the hooks with the body tissue. For example, as discussed below, the action of the hooks unfolding during implantation may compress the plugging device against the body tissue such that tissue growth is encouraged.

The plugging device is configured to assist in sealing an entry site of the hooks in the body tissue when the anchor is implanted in the body tissue. The plugging device is configured to assist in plugging entry sites of the hooks in the body tissue when the anchor is implanted in the body tissue. The sealing and/or plugging may be provided due to compression of the plugging device against the body tissue. The sealing and/or plugging may be provided due to a suction of the plugging device against the body tissue. The compression and/or suction may be provided by a fluid pressure differential across the body tissue. Additionally or alternatively, the compression may be provided by the action of the hooks unfolding during implantation.

It will be appreciated that, whilst the anchor configuration may be suitable for e.g. cardiac treatments such as implanting an artificial chordae line for repair of chordae tendineae, the anchor configuration disclosed herein may also be suitable for other treatments within the body.

For example, the anchor configuration may comprise or be in combination with an implantable component such as a stent, valve, or other device configured to be implanted in body tissue. The implantable component could be a slow-release drug or compound used in a treatment of the body. The implantable component may be joined to a body portion of the anchor by a line or other suitable solution, such as via brazing, welding, bonding or the like. Thus when implantable components are implanted within body tissue using the anchor configuration of the present aspect, the plugging device may plug and/or seal entry sites of the hooks of the anchor and/or may encourage tissue ingrowth around the anchor and the plugging device, such that healing of the body tissue is better facilitated following implantation of the anchor configuration comprising and/or in combination with the implantable component.

Alternatively, the anchor configuration could be used without being in combination with a further component. For example, the anchor configuration may comprise an anchor formed of a radiopaque material, and as such may be used as a marker during imaging of the body. Additionally or alternatively, the anchor configuration may be used to staple regions of body tissue together. The use of the anchor configuration of the first aspect is therefore not intended to be restricted to any specific treatment or function within the body, but may perform or assist in a variety of treatments.

The entry site may comprise a hole pierced into the body tissue that may result from engagement of each hook with the body tissue. The hole may be in the form of a channel or passageway through the tissue. The plugging device may be configured to abut or contact the body tissue surrounding the entry sites where the hooks engage the body tissue. This contact is configured to seal an entry site(s) at the location where the body tissue is first engaged. The plugging device may block an aperture of the entry sites, such as a channel and/or passageway into or through the body tissue. Sealing the entry site of the hooks in the body tissue may prevent or impede flow, and in particular high velocity flow, from passing through the openings at the hook entry sites.

A portion of the plugging device encircling the at least one hook encircles the respective hook. The portion of the plugging device encircling the hook may surround a circumference, perimeter and/or circumferential extent of the respective hook. If more than one hook is encircled, i.e. at least two of the hooks are each encircled by the plugging device, each hook may be provided with at least one encircling portion of the plugging device in contact with an entry site, i.e. the narrow channel and/or passageway, where it engages the body tissue such that a flow of fluid through the site of implantation is impeded or prevented.

In some optional arrangements, at least one of the number of hooks may not have a portion of the plugging device encircling it. As such, only some (i.e. the at least one) of the hooks may be encircled by the plugging device. The provision of one or more portions of the plugging device encircling at least some of the hooks may provide a sufficient blocking and/or sealing effect over the narrow channels and/or passageways created by the hooks with a portion of the plugging device encircling them, such that the effects of high velocity jets and/or fluid flow impeding tissue growth are still sufficiently reduced.

All of the hooks may be in contact with at least a portion of the plugging device. Whilst the hook may or may not have at least a respective portion of the plugging device encircling it, a portion of the plugging device may be at least in contact with a circumferential portion and/or at least an arc or side of the circumferential extent of the hook.

The plugging device may surround some or all of at least two of the hooks. The plugging device may be in contact with a circumferential portion and/or arc or side of the circumference of the hook. The plugging device may not comprise a respective portion to encircle each hook, but may surround each hook such that both hooks are encircled by a circumferential extent of the plugging device. The plugging device along with the anchor may therefore be configured to provide an overall sealing effect around the entry sites formed by each hook during engagement of the hooks in the body tissue.

The plugging effect achieved by the portions of the plugging device encircling the at least one hook may be used in addition to a plugging effect generally achieved by the plugging device encompassing and/or surrounding the circumferential extent of the anchor as a whole. For example, the at least one hook with a respective portion of the plugging device encircling it may also have a larger portion of the plugging device surrounding and/or encircling them which captures all the hooks. The portions of the plugging device encircling the at least one hook may provide a localised sealing effect at the entry site where the hook engages the body tissue. The portion of the plugging device surrounding and/or encircling all the hooks may provide greater security of the plugging device and/or may provide an additional sealing effect over a captured area, which includes all the entry sites where each of the at least one hooks engages the body tissue.

In addition to or alternatively to the portion of the plugging device encircling all of the number of hooks, the plugging device may comprise a portion encircling two or more of the hooks. The portion encircling the two or more hooks may capture each of the two or more hooks.

The anchor may comprise an anchor body. The hooks may extend from a base of the anchor body. The anchor body may be a tubular body. The hooks may extend distally from the anchor body. Where the hooks diverge from the anchor body may be regarded as the base of the anchor body. The base of the anchor body may be a part of the anchor body which is configured to abut body tissue upon implantation of the anchor in body tissue. The anchor body may be a hollow body. The anchor body may be a prism or a cylinder. The faces of the prism or cylinder may be open at each end, such that the anchor body is a hollow prism or hollow cylinder.

The anchor body may define a central axis. In the folded position the hooks may extend substantially parallel to and displaced from the central axis. In the unfolded position the hooks may extend in a substantially perpendicular direction from the central axis.

The base may be located at a distal end of the anchor body. Tips of the hooks for piercing the body tissue during engagement of the hooks with the body tissue may be at a distal end of the hooks.

Distal will be understood to be a direction of the anchor in which the anchor is to be implanted in body tissue, i.e. in the folded position it is the direction in which the hooks may extend from the anchor body. Proximal will be understood to be a direction opposite to the distal direction.

When the hooks are not constrained by the application of a constraining force in the folded position, the hooks will return to the unfolded position. As this occurs during implantation of the anchor in body tissue, the hooks may display a springback effect owing to the elastic properties of the anchor. The unfolding of the hooks may act to pull the anchor further through the body tissue during implantation. The unfolding of the hooks may subsequently pull and/or compress the plugging device against the body tissue as it pulls the anchor through the body tissue.

The anchor body may be configured to compress the plugging device against the body tissue when the hooks are in the unfolded position and the anchor is implanted in body tissue. Compressing the plugging device against the body tissue may strengthen the sealing effect of the plugging device. The compressive force applied by the anchor body to the plugging device may be a reaction force to the springback effect owing to the elastic properties of the anchor. That is, the compressive force applied by the anchor body to the plugging device may be generated by the unfolding action of the hooks pulling the anchor through the body tissue.

Additionally/alternatively, the compressive force applied by the anchor body to the plugging device may be the result of a fluid pressure on the side of the body tissue the anchor body is located. The fluid pressure may exert a force over the anchor body and/or the plugging device in the direction of implantation, such that the plugging device is pressed against the body tissue. For example, if the anchor is implanted in a mitral leaflet from a ventricular side through to an atrial side of the leaflet, a fluid pressure generated in the ventricular side due to contraction of the heart may exert a force on the anchor body and/or on the plugging device in a direction into the body tissue, i.e. generally towards the leaflet. The higher blood pressure at the ventricular side and lower blood pressure at the atrial side may result in compression of the plugging device against the body tissue, and/or a seal being formed by suction pressure at the entry site if tissue regrowth has not yet blocked pathways along the hooks through the leaflet.

The plugging device may be located at a base of the anchor. Where the hooks may extend from the base of the anchor may be regarded as a base of the hooks. The plugging device may be located around/adjacent to the base of the hooks and the base of the anchor body. The plugging device may be configured to be located between the body tissue and the anchor body when the anchor is implanted in body tissue.

At least one of the hooks has a portion of the plugging device encircling it, and thus this portion (as well as optionally other portions, e.g. when at least two of the hooks are encircled by the plugging device, or more) of the plugging device may be located between the body tissue and the body of the anchor when the anchor is implanted in the body tissue. When in the unfolded position and when implanted in body tissue, the hooks may therefore exert a force which pulls the body of the anchor against the body tissue, and in turn compress this portion against the body tissue when the hooks are in the unfolded position.

At least one portion of the plugging device may be configured to occupy a gap and/or channel between the base of the anchor and the body tissue when the anchor is implanted in body tissue. The at least one portion configured to occupy the gap and/or channel between the anchor and the body tissue may be wrapped around the body and/or base of the anchor. The at least one portion configured to close the gap and/or channel between the anchor and the body tissue may encircle one or more of the hooks. The at least one portion configured to occupy the gap and/or channel between the base of the anchor and the body tissue may help stabilise the anchor when implanted in body tissue. Additionally or alternatively, the at least one portion configured to occupy the gap and/or channel between the base of the anchor and the body tissue may help prevent a flow of fluid between the base of the anchor and the body tissue. That is, the at least one portion configured to occupy the gap and/or channel between the base of the anchor and the body tissue may be configured to seal the gap and/or channel between the base of the anchor and the body tissue.

The at least one portion configured to occupy a gap and/or channel between the base of the anchor and the body tissue may substantially be part of and/or about the portions encircling each hook. The at least one of the portions configured to seal a gap and/or channel between the base of the anchor and the body tissue may, in combination with the portions encircling each of the at least one or more hooks, comprise a sealing surface. The sealing surface may be configured to contact an area of body tissue in which the anchor is to be implanted in body tissue. The sealing surface may contact all entry sites where the hooks engage the body tissue, and may contact an overall area and/or perimeter capturing all the narrow channels and/or passageways.

The plugging device may comprise one or more compressible regions extending along and encircling a respective hook. The compressible region may be a portion of the plugging device encircling at least one of the encircled hooks. The compressible region may comprise a tubular portion and/or a plurality of loops encircling the at least one hook, and the tubular portion and/or the plurality of loops may extend along a length of the hook(s) whilst leaving tips of the hook(s) exposed. The compressible region may be generally helical.

The compressible region may be configured to at least partially pass through the body tissue when the anchor is implanted in the body tissue, i.e. when the tips of the hook(s) pierce the body tissue. The compressible region may be configured to collapse and/or compress around the body tissue, such that a first portion of the compressible region is formed on one side of the entry site (i.e. where the tips of the anchor pierce through to) and a second portion of the compressible region (i.e. where the anchor body remains). As such the plugging device may be configured to provide a plugging/sealing effect, and/or facilitate the ingrowth of tissue, on both sides of the entry site.

The anchor body may be a tubular body. At least part of the plugging device may be located within the tubular body. The tubular body may be hollow, such that at least part of the plugging device may be located within the tubular body.

By providing at least part of the plugging device within the tubular body, the plugging device may be at least in part protected flow any flow of fluid to which the anchor may be subjected, for example, blood during the cardiac cycle. The integrity of the plugging device may therefore be maintained, such that the plugging device may more securely combine with the anchor.

The anchor body may comprise at least two threading holes. The threading holes may provide a closed aperture through which a line or a stent, valve or the like may be joined to the anchor.

The threading holes may be evenly distributed uniformly around the anchor body. The threading holes may be located equidistant between adjacent hooks of the anchor, relative to where the hooks extend from the anchor body. The threading holes may be located proximally from the base of the anchor body. The threading holes may be at a midpoint of an axial length of the anchor body.

If the anchor comprises only two hooks, the hooks may be disposed opposite each other, e.g. 180 degrees from one another around a circumference of the anchor body, or extending from opposite sides of the walls of the anchor body. The anchor may comprise two threading holes. Each threading hole may be disposed opposite each other, e.g. 180 degrees from one another around a circumference of the anchor body, or extending from opposite sides of the walls of the anchor body. The threading holes may each be disposed 90 degrees from the anchors around a circumference of the anchor body, or extending from opposite sides of the walls of the anchor body.

Each of the portions of the plugging device encircling each of the at least one hook may be configured to provide a first sealing surface around each respective entry site of the at least one hook. The portion of the plugging device surrounding the anchor as a whole may be configured to provide a second sealing surface capturing each of the entry sites of each of the hooks.

As discussed above and discussed herein, a portion of the plugging device encircling the hook is configured to seal an entry site between the hook and the body tissue when the hook engages the body tissue. The seal may be formed due to a plugging effect of the portion against the entry site of the hook when the anchor is implanted in body tissue, and/or by providing a sealing surface analogous to an O-ring which impedes a flow of fluid. Each hook which has a portion of the plugging device encircling it may be considered as having a first sealing surface around it.

For example, the seals may be located on a high-pressure side of the body tissue, with the entry sites extending from the high-pressure side to a low-pressure side. The pressure differential across the body tissue, and hence the entry sites, may provide a suction and/or suction force which pulls the seals towards the body tissue. This may provide the plugging effect and/or strengthen the plugging effect when there has not yet been any tissue regrowth across pathways through the body tissue. This reduces or prevents the flow of fluid through such pathways, and hence avoids allows better tissue regrowth, reducing any inhibition of tissue regrowth that may occur due to flow of fluid.

In addition to the first sealing surface provided around at least one of the hooks, the plugging device may be configured to provide a second sealing surface capturing each of the entry sites of each of the hooks. The second sealing surface may be formed of a portion of the plugging device encircling and/or surrounding each of the hooks, or may be formed from portions of the plugging device which in combination form a sealing surface surrounding an outer perimeter around each of the hooks. The second sealing surface may be analogous to an O-ring surrounding an area substantially equal to a cross-sectional area of the anchor at the base of the anchor. The second sealing surface may therefore provide a sealing surface which captures each of the entry sites of the hooks.

The provision of two sealing surfaces (i.e. the one or more first sealing surfaces and the second sealing surface) may impede and/or prevent a flow of fluid through the entry sites of the hooks when the anchor is implanted in body tissue. This may in turn encourage tissue ingrowth around the anchor and thus improve the tensile strength of the anchor when implanted in body tissue.

The second surface may additionally provide a failsafe sealing surface, in the event that any one of the first sealing surfaces is not formed when the anchor is implanted in the body tissue. This may improve the reliability of the anchor in combination with a plugging device in impeding and/or preventing high velocity jets and/or a flow of fluid flowing through the entry sites.

The number of hooks may be a first hook and a second hook, and at least two of the hooks may be encircled by the plugging device. Thus the at least one hook may be two hooks, which may be the first and the second hooks. The plugging device may comprise at least one portion around a first hook. The plugging device may comprise at least one portion around the second hook. The at least one portion of the plugging device encircling the first hook and the at least one portion of the plugging device encircling the second hook may be the at least one portion of the plugging device encircling each of the at least two hooks.

The portions of the plugging device encircling the first and second hooks respectively may be considered as each providing a/the first sealing surface. The portions around the first and second hooks, in combination with the portion of the plugging device surrounding the anchor/encircling each of the first and second hooks may be considered as providing the second sealing surface.

The plugging device may be formed of a biocompatible material. The biocompatible material may be ePTFE.

The hooks may be formed with openings along their lengths. The openings preferably do not accommodate any part of the knotting configuration.

By adding openings in the anchor hooks a larger width hook can be used thereby increasing the holding strength while still allowing significant deformation between the folded and unfolded position without any plastic deformation. The increased surface area of the larger width hook also aids in spreading the distribution of forces. The openings may also enhance healing by allowing tissue to growing in between the slits, making a more reliable connection between the anchor and the tissue over time, rather than the tissue forming a "sock" that may be pulled out more easily, as would be the case with a solid hook.

In some examples the openings in the hooks include multiple holes (such as multiple holes of with a diameter of about 0.2-0.4mm), with these openings connected with a suture, wherein a single length of suture passes through several of the multiple holes, or all of the multiple holes. The suture may be knotted at each hole. The suture may for example be a Dyneema suture (or other similar suture, such as Dacron). Elastic materials such as nitinol can be prone to fatigue fracturing during high cyclic loads, including the cyclic loads that will arise from a beating heart. By the use of a suture through multiple holes it is possible to add a failsafe to the anchor hooks. If the hooks of the anchor break then the anchor is still kept together by the suture, which reduces risk for embolism while also providing extra time for ingrowth of tissue. Thus, even if an anchor breaks at an early stage then it will not embolise, and it will still be able to hold some force, as the expanded anchor will be too large to be pulled through its entry hole even if one or more hooks suffer a fracture. The use of a suture in this way will also make more "openings" for tissue to grow through. The multiple holes may be circular holes made in addition to other openings in the hooks, such as being made in addition to slits as discussed below.

As an alternative to the use of a suture threaded through the openings the anchor may include an overmolding, which may be provided about the entire anchor excluding the sharp tips of the hooks could be possible. A suitable material for such an overmolding is ePTFE. Another alternative is to use a woven fabric pouch that encloses the anchor. Both of these solutions would keep the anchor from embolising if there is a fracture in the anchor. The use of ePTFE also gives the added benefit of tissue ingrowth.

The anchor may be formed from a tube that is cut to provide tines extending from one end of the tube, with these tines then being curved and heat set to form the hooks. The end of the tube from which the hooks extend may be the anchor body. Openings can be cut into the tines before or after they are curved, but typically before in order that there is only one cutting stage. An added benefit of the use of openings in relation to this construction is that small diameter tubing becomes more pliable with an opening in the centre, since the arc of the tube is divided into two smaller arcs. As a result a wider section of a narrow tube can be safely utilized for making the tines which again gives additional strength. As a result of the increased holding force and increased pliability the anchor hooks are subjected to less fatigue load which in turn makes the implant last longer.

The openings may be formed as a series of holes, or as slits extending along the length of the tines to thereby extend along the curves of the hooks. A benefit of the use of slits is that each hook consists of two "legs" meaning that a fracture in one of the "legs" does not mean it will embolise, and the anchor will still be held in place by the other leg. At the same time the new "V" shape leg will highly likely grow into tissue more effectively than a straight "broken" hook without any slit or other openings, further reducing the danger of embolism.

The openings may include several smaller slits in line or have different types of pattern (zig-zag, barbed or wave pattern are examples). Along the length of the hooks, small holes with different patterns may be made, either instead of slits or in addition to slits. This can provide additional holding force, when tissue grows through the holes. It can also allow for a suture to be threaded through the hooks for added safety in the event of a fracture as discussed above. The slits may also be extended beyond the ends of the hooks where they join into the base of the anchor, which may be a tube shaped part as discussed above, thereby making the base more flexible as well. In some examples the slits may be cut as a single laser track. Circular openings can be added to the ends of such a cut to prevent high strain points.

In one example the anchor is cut from tubing made of an elastic metal, such as nitinol. Laser cutting may be used. This can involve cutting tines as discussed above, which can be heat set into curves. The anchor may be heat treated and/or electropolished. Chamfered edges may be introduced to the anchor on certain parts before the anchor is electropolished. The openings could contain a barbed or wave profile along edges of the openings, e.g. along edges of slits. Where slits are used the slotted hooks can be heat set in a configuration where they have increased distance when deployed. A barbed profile can then be concealed when the hooks are straight (barbs are facing towards one another). With this example, when the anchor comes to a non-constrained configuration then the slits move apart and the barb profile is engaged.

The anchor may be a leaflet anchor for use in the heart. The anchor configuration may comprise a line in combination with the anchor, and the line may be an artificial chordae line. The body tissue may be a mitral leaflet. The anchor in combination with the line may therefore be a cardiac surgical device comprising the anchor and line as a leaflet anchor and artificial chordae line. The overall function of the leaflet anchor in combination with the artificial chordae line may be similar to the leaflet anchor in combination with a line of WO2016/042022. However, it will be appreciated that the features and structure of the anchor configuration herein provides a departure from the prior art, adding benefits not found with the anchor device proposed in WO2016/042022.

In various aspects the invention extends to the use of the anchor configurations comprising anchors in combination with lines described above with catheter devices, and in particular to the use of those devices during a procedure for implanting an artificial chordae line into the heart. Further, the invention extends to the manufacture of the anchor configurations comprising and/or in combination with lines, stents, valves and the like as described above, including various method steps of forming the anchors such as laser cutting from tubes. For any of the anchors of the anchor configurations, or other laser cut parts discussed herein chamfered edges may be introduced before the laser cut part (e.g. anchor) is electropolished. Alternatively, the chamfered edges may be introduced by electropolish. The features of the first aspect and other optional features discussed above may be combined with the other aspects discussed below, with the anchors of those other aspects hence having an anchor configuration comprising an anchor and a plugging device accordance with the first aspect.

As mentioned above, the plugging device is a line, the line in combination with the anchor. The anchor configuration can hence also be regarded as an anchor for implantation in body tissue in combination with the line. The line is joined to the anchor by a knotting configuration comprising a plurality of loops around the anchor; and the at least one hook may be encircled by at least one loop of the plurality of loops. In some embodiments, at least two of the hooks of the anchor may be each encircled by at least one loop of the plurality of loops. The knotting configuration may be regarded as the specific portion of the line which comprises the features of the plugging device.

The line may consist of only the knotting configuration, or alternatively the line may continue from and extend from the knotting configuration, the knotting configuration forming the plugging device and a joining point of the line to the anchor. The line may be an artificial chordae line or the artificial chordae line as discussed above, and hence the plugging device may be regarded as a specific portion of the artificial chordae line comprising the features of the plugging device.

Thus, viewed from a second aspect the invention provides an anchor for implantation in body tissue in combination with a line. The anchor comprises: a number of hooks for engagement with the body tissue and having a folded and unfolded position, wherein the anchor is made of an elastic material such that it can be elastically deformed into the folded position by application of a constraining force, and will return to the unfolded position when no constraining force is applied; wherein the line is joined to the anchor by a knotting configuration comprising a plurality of loops around the anchor; wherein at least one of the hooks is encircled by at least one loop of the plurality of loops; and wherein the at least one hook is encircled by at least one loop of the plurality of loops.

The anchor in combination with a line of the second aspect may have one or more features corresponding to the features of the anchor configuration of the second aspect. This the above description of the anchor configuration of the first aspect, including but not limited to all technical advantages and alternative embodiments, may be equally applicable to the anchor in combination with a line of the first aspect.

Whilst discussed in relation to an anchor in combination with a line wherein at least one of the hooks is encircled by at least one loop of the plurality of loops, it will be appreciated that, in accordance with the first aspect, that the description below may be equally applicable to an anchor in combination with a line wherein at least two of the hooks are each encircled by at least one loop of the plurality of loops.

Upon implantation of a conventional anchor in body tissue, the hooks engage the body tissue via entry sites. In situations where the hooks completely pass through the tissue during engagement, a narrow channel and/or passageway may result around each hook. Where the tissue is adjacent to a fluid of a sufficient fluid pressure, for example blood during contraction of the heart in the cardiac cycle, the fluid may be forced through the entry sites, such as the narrow channel and/or passageway, and produce a high velocity jet. For example, if the anchor was implanted in a mitral valve of a heart, the circulation of blood through the mitral valve may be of sufficient pressure such that a high velocity jet of blood is forced through each location where a hook of the anchor engages the mitral valve. A flow of blood to each location where a hook of the anchor engages the mitral valve to produce the high velocity jets may pass between the anchor and the body tissue and/or may drive the anchor away from the body tissue it is implanted in. This high velocity jet and/or a flow of blood producing the high velocity jet may impede tissue growth at the site of implantation in body tissue and thus weaken the resultant tensile strength of the implanted anchor.

By providing a plurality of loops around the anchor in the knotting configuration which joins the line to the anchor, the loops of line may close openings through the body tissue at entry sites produced by the hooks during engagement with body tissue. The loops may act to increase a cross-sectional area of the anchor where they are looped, such that flow through the entry sites is impeded. At least impeding the flow may reduce movement of the implanted anchor due to flow through the entry sites. Impeding or preventing the flow of fluid through the hook entry sites may facilitate tissue growth around the site where the anchor is implanted in body tissue and increase the overall tensile strength of the anchor when implanted in body tissue. In an example where the anchor is implanted in a leaflet of a mitral valve, if a main body of the anchor and the line is on the high pressure side then the blood pressure can push the anchor and/or the looped line toward the entry sites of the hooks, hence forming a strong barrier with a sealing effect preventing unwanted blood flow through the entry sites.

Requiring that at least one of the hooks has at least one loop of the plurality of loops wrapped around them may provide localised sealing, or impeding of flow, to the hooks which are encircled by the line. This may help stabilise these hooks, and reduce motion of the anchor overall when implanted in the body tissue. As the openings at the entry sites originate due to the engagement of the hooks with the body tissue, providing at least one loop around at least one of the hooks may aid sealing of the entry sites of the at least one hook, as well as also aiding tissue re-growth and/or aiding sealing of adjacent entry sites. The localised sealing and/or impeding effect provided by the loops around each of these hooks against flow through the entry sites may be in addition to the general impeding of flow provided by the plurality of loops.

At least two of the hooks may be encircled by at least one loop of the plurality of loops. There may be at least one loop around each of the number of hooks. For example, an anchor may have only two hooks with at least one loop around each hook, or some other number of hooks with at least one loop around each hook. The use of a loop round each hook can give a similar treatment for each entry site of the hooks.

Additionally, the provision of a plurality of loops in the knotting configuration joining the anchor to the line may facilitate tissue ingrowth around the anchor, such that the anchor is better secured to the body tissue it is implanted in. The line may provide additional surface area for tissue to grow around. The line may additionally be of a biocompatible material which encourages tissue ingrowth. It will be appreciated that the plurality of loops may encourage tissue ingrowth regardless of whether the anchor is subject to high velocity jets and/or fluid flows resulting from engagement of the hooks with the body tissue. For example, as discussed below, the action of the hooks unfolding during implantation may compress the knotting configuration/the plurality of loops against the body tissue such that tissue growth is encouraged.

The plurality of loops may be configured to assist in sealing an entry site of the hooks in the body tissue when the anchor is implanted in the body tissue. The plurality of loops may be configured to assist in plugging entry sites of the hooks in the body tissue when the anchor is implanted in the body tissue. The sealing and/or plugging may be provided due to compression of the plurality of loops against the body tissue. The sealing and/or plugging may be provided due to a suction of the plurality of loops against the body tissue. The compression and/or suction may be provided by a fluid pressure differential across the body tissue. Additionally or alternatively, the compression may be provided by the action of the hooks unfolding during implantation.

The entry site may comprise a hole pierced into the body tissue that may result from engagement of each hook with the body tissue. The hole may be in the form of a channel or passageway through the tissue. The plurality of loops may be configured to abut or contact the body tissue surrounding the entry sites where the hooks engage the body tissue. This contact may seal the entry sites at the location where the body tissue is first engaged. The plurality of loops may block an aperture of the entry sites, such as a channel and/or passageway into or through the body tissue. Sealing the entry site of the hooks in the body tissue may prevent or impede flow, and in particular high velocity flow, from passing through the openings at the hook entry sites.

Each of the at least one loops wrapped around the at least one hook encircles the respective hook. The loops wrapped around the hook may surround a circumference, perimeter and/or circumferential extent of the respective hook. If more than one hook is encircled, i.e. at least two of the hooks are each encircled by at least one loop of the plurality of loops, each hook may be provided with at least one loop of line in contact with an entry site, i.e. the narrow channel and/or passageway, where it engages the body tissue such that a flow of fluid through the site of implantation is impeded or prevented.

Two or more loops may be wrapped around the at least one hook. The two loops may be stacked on top of one another. In other words, the loops may not overlap one another or extend radially outwards from the hook by more than the diameter of the line. The loops may wrap around the respective hook in a coiled fashion. The two or more loops may be coiled around each of the at least two hooks. The direction of stacking and/or coiling may be along a length of the hooks.

The knotting configuration may be tensioned to tighten it. Tensioning the knotting configuration may ensure that each of the plurality of loops may be taut around the anchor and around the at least one hook thereof, such that they comprise no slack along their length. The knotting configuration may be tensioned to at least 4.5N. The knotting configuration may be tensioned to at least 5N. The knotting configuration may be tensioned to at least 5.5N. Such tensioned forces may ensure that the knotting configuration is tightened and is taut without snapping and/or damaging the line.

In some optional arrangements, at least one of the number of hooks may not have a loop of the plurality of loops wrapped around it. As such, only some (i.e. the at least one) of the hooks may have a loop wrapped around it. The provision of a loop around at least some of the hooks may provide a sufficient blocking and/or sealing effect over the narrow channels and/or passageways created by the hooks with a loop wrapped around them, such that the effects of high velocity jets and/or fluid flow impeding tissue growth are still sufficiently reduced.

All of the hooks may be in contact with at least a portion of the line. Whilst the hook may or may not have at least a loop of line wrapped around it, a portion of the line may be at least in contact with a circumferential portion and/or at least an arc or side of the circumferential extent of the hook.

The portion of the line in contact with the hook may be adjacent to a loop of line wrapped around an adjacent hook, such that the portion of the line is effectively a continuation of the loop of line wrapped around the adjacent hook. Accordingly, the portion of line may be configured to provide a combined sealing effect with adjacent loops of line. Additionally or alternatively, the portion of the line in contact with the hook may be in contact with a portion of line in contact with an adjacent hook.

A loop of the plurality of loops may be wrapped around at least two of the hooks. A portion of the line wrapped around the at least two hooks may be in contact with a circumferential portion and/or arc or side of the circumference of the hook. The loop may not be wrapped around each hook such that each hook is encircled, but may be wrapped around each hook such that both hooks are captured by the loop. The loop wrapped around at least two of the hooks may be configured to provide an overall sealing effect around the entry sites formed by each hook during engagement of the hooks in the body tissue.

The loop wrapped around at least two of the hooks may be used in combination with a larger loop wrapped around all of the number of hooks. For example, the at least two hooks each with at least one loop wrapped around them may also have a larger loop wrapped them which captures all the hooks. The loops wrapped around each hook may provide a localised sealing effect at the entry site where the hook engages the body tissue. The loop wrapped around all the hooks may provide greater security of the knotting configuration and/or may provide an additional sealing effect over a captured area, which includes all the entry sites where each of the at least two hooks engages the body tissue.

In addition to or alternatively to the loop wrapped around all of the number of hooks, the plurality of loops may comprise a loop wrapped around two or more of the hooks. The loop wrapped around the two or more hooks may capture each of the two or more hooks.

The knotting configuration may be considered as comprising at least one knot, as well as the plurality of loops. The plurality of loops may form part of the knot, or may be separate from the knot. The knotting configuration may tighten the line around the anchor such that it remains in place when the line and the anchor are subjected to tensile forces in opposing directions. The knotting configuration is preferably formed only from the single line provided in combination with the anchor.

The knotting configuration may comprise at least two knots. At least one of the knots may comprise at least one of the plurality of loops. Two of the knots may be located each at a proximal and a distal end of the knotting configuration. In other words, a start and an end of the knotting configuration may be defined by a knot at each end. The knots at each end may surround and/or capture the anchor and the plurality of loops such that the anchor is joined to the line.

The anchor may comprise an anchor body. The hooks may extend from a base of the anchor body. The anchor body may be a tubular body. The hooks may extend distally from the anchor body. Where the hooks diverge from the anchor body may be regarded as the base of the anchor body. The base of the anchor body may be a part of the anchor body which is configured to abut body tissue upon implantation of the anchor in body tissue. The anchor body may be a hollow body. The anchor body may be a prism or a cylinder. The faces of the prism or cylinder may be open at each end, such that the anchor body is a hollow prism or hollow cylinder.

The anchor body may define a central axis. In the folded position the hooks may extend substantially parallel to and displaced from the central axis. In the unfolded position the hooks may extend in a substantially perpendicular direction from the central axis.

The base may be located at a distal end of the anchor body. The line may substantially extend from a proximal end of the anchor body. Tips of the hooks for piercing the body tissue during engagement of the hooks with the body tissue may be at a distal end of the hooks.

Distal will be understood to be a direction of the anchor in which the anchor is to be implanted in body tissue, i.e. in the folded position it is the direction in which the hooks may extend from the anchor body. Proximal will be understood to be a direction opposite to the distal direction. The line may substantially extend proximally from the knotting configuration when in combination with the anchor.

When the hooks are not constrained by the application of a constraining force in the folded position, the hooks will return to the unfolded position. As this occurs during implantation of the anchor in body tissue, the hooks may display a springback effect owing to the elastic properties of the anchor. The unfolding of the hooks may act to pull the anchor further through the body tissue during implantation. The unfolding of the hooks may subsequently pull and/or compress the plugging device against the body tissue as it pulls the anchor through the body tissue.

The anchor body may be configured to compress the plurality of loops against the body tissue when the hooks are in the unfolded position and the anchor is implanted in body tissue. Compressing the plurality of loops against the body tissue may strengthen the sealing effect of the plurality of loops. The compressive force applied by the anchor body to the plurality of loops may be a reaction force to the springback effect owing to the elastic properties of the anchor. That is, the compressive force applied by the anchor body to the plugging device may be generated by the unfolding action of the hooks pulling the anchor through the body tissue.

Additionally/alternatively, the compressive force applied by the anchor body to the plurality of loops may be the result of a fluid pressure on the side of the body tissue the anchor body is located. The fluid pressure may exert a force over the anchor body and/or the line in the direction of implantation, such that the plurality of loops are pressed against the body tissue. For example, if the anchor is implanted in a mitral leaflet from a ventricular side through to an atrial side of the leaflet, a fluid pressure generated in the ventricular side due to contraction of the heart may exert a force on the anchor body and/or on the looped line in a direction into the body tissue, i.e. generally towards the leaflet. The higher blood pressure at the ventricular side and lower blood pressure at the atrial side may result in compression of the plurality of loops against the body tissue, and/or a seal being formed by suction pressure at the entry site if tissue regrowth has not yet blocked pathways along the hooks through the leaflet.

The knotting configuration may be located at a base of the anchor. Where the hooks may extend from the base of the anchor may be regarded as a base of the hooks. The plurality of loops may be located around the base of the hooks and the base of the anchor body. The plurality of loops may be configured to be located between the body tissue and the anchor body when the anchor is implanted in body tissue.

At least one of the hooks has a loop wrapped around and encircling it, and thus this loops (as well as optionally other loops, e.g. of the respective hook or of other hooks) may be located between the body tissue and the body of the anchor when the anchor is implanted in the body tissue. When in the unfolded position and when implanted in body tissue, the hooks may therefore exert a force which pulls the body of the anchor against the body tissue, and in turn compress this loop against the body tissue when the hooks are in the unfolded position.

At least one of the plurality of loops may be configured to occupy a gap and/or channel between the base of the anchor and the body tissue when the anchor is implanted in body tissue. The at least one loop configured to occupy the gap and/or channel between the anchor and the body tissue may be wrapped around the body and/or base of the anchor. The at least one loop configured to close the gap and/or channel between the anchor and the body tissue may be wrapped around one or more of the hooks. The at least one of the plurality of loops configured to occupy the gap and/or channel between the base of the anchor and the body tissue may help stabilise the anchor when implanted in body tissue. Additionally or alternatively, the at least one of the plurality of loops configured to occupy the gap and/or channel between the base of the anchor and the body tissue may help prevent a flow of fluid between the base of the anchor and the body tissue. That is, the at least one of the plurality of loops configured to occupy the gap and/or channel between the base of the anchor and the body tissue may be configured to seal the gap and/or channel between the base of the anchor and the body tissue.

The at least one of the plurality of loops configured to occupy a gap and/or channel between the base of the anchor and the body tissue may substantially be in contact with and/or abut the loops wrapped around each hook. The at least one of the plurality of loops configured to seal a gap and/or channel between the base of the anchor and the body tissue may, in combination with the loops wrapped around each of the at least one or more hooks, comprise a sealing surface. The sealing surface may be configured to contact an area of body tissue in which the anchor is to be implanted in body tissue. The sealing surface may contact all entry sites where the hooks engage the body tissue, and may contact an overall area and/or perimeter capturing all the narrow channels and/or passageways.

The knotting configuration may comprise a plurality of compressible loops extending along and encircling a respective hook. The plurality of compressible loops may be configured to be compress, bunch and/or slide up and/or down the hook which they encircle. The plurality of compressible loops may be separate to, or may be the same as, the plurality of loops encircling at least one of the hooks.

The plurality of compressible loops may be configured to at least partially pass through the body tissue when the anchor is implanted in the body tissue, i.e. when the tips of the hooks pierce the body tissue. The plurality of compressible loops may be configured to collapse and/or compress around the body tissue, such that a first portion of the compressible region is formed on one side of the entry site (i.e. where the tips of the anchor pierce through to) and a second portion of the compressible region (i.e. where the anchor body remains). As such the knotting configuration may be configured to provide a plugging./sealing effect, and/or facilitate the ingrowth of tissue, on both sides of the entry site.

The anchor body may be a tubular body. At least part of the knotting configuration may be located within the tubular body. The tubular body may be hollow, such that at least part of the knotting configuration may be located within the tubular body.

By providing at least part of the knotting configuration within the tubular body, the plurality of loops not within the tubular body may form a relatively flat sealing surface with which to contact the body tissue. A number of loops and/or knots which may provide the tightening properties of the knotting configuration may therefore be displaced from the plurality of loops which may be configured to seal an entry site of the hooks, such that a more secure seal may be provided by the plurality of loops.

By providing at least part of the knotting configuration within the tubular body, the knotting configuration may be at least in part protected flow any flow of fluid to which the anchor may be subjected, for example, blood during the cardiac cycle. The integrity of the knotting configuration may therefore be maintained, such that the line is more securely joined to the anchor.

A first knot of the knotting configuration may be located within the tubular body. The first knot may be located at a proximal end of the tubular body. The line may extend proximally from the first knot out of the tubular body. The rest of the knotting configuration may be located distally from the first knot.

The anchor body may comprise at least two threading holes. The threading holes may accommodate at least part of the knotting configuration. The threading holes may be located on the wall(s) of the anchor body. The threading holes may provide a closed aperture through which the knotting configuration may be joined to the anchor.

The threading holes may be evenly distributed uniformly around the anchor body. The threading holes may be located equidistant between adjacent hooks of the anchor, relative to where the hooks extend from the anchor body. The threading holes may be located proximally from the base of the anchor body. The threading holes may be at a midpoint of an axial length of the anchor body.

If the anchor comprises only two hooks, the hooks may be disposed opposite each other, e.g. 180 degrees from one another around a circumference of the anchor body, or extending from opposite sides of the walls of the anchor body. The anchor may comprise two threading holes. Each threading hole may be disposed opposite each other, e.g. 180 degrees from one another around a circumference of the anchor body, or extending from opposite sides of the walls of the anchor body. The threading holes may each be disposed 90 degrees from the anchors around a circumference of the anchor body, or extending from opposite sides of the walls of the anchor body.

Each of the at least one loops encircling the at least one hook may be configured to provide a first sealing surface around each respective entry site of the at least one hook. The plurality of loops may be configured to provide a second sealing surface capturing each of the entry sites of each of the hooks.

As discussed above and discussed herein, a loop of line wrapped around the hook is configured to seal an entry site between the hook and the body tissue when the hook engages the body tissue. The seal may be formed due to a plugging effect of the line against the entry site of the hook when the anchor is implanted in body tissue, and/or by providing a sealing surface analogous to an O-ring which impedes a flow of fluid. Each hook which has at least one loop of line wrapped around it may be considered as having a first sealing surface around it.

For example, the seals and the plurality of loops may be located on a high-pressure side of the body tissue, with the entry sites extending from the high-pressure side to a low-pressure side. The pressure differential across the body tissue, and hence the entry sites, may provide a suction and/or suction force which pulls the seals towards the body tissue. This may provide the plugging effect and/or strengthen the plugging effect when there has not yet been any tissue regrowth across pathways through the body tissue. This reduces or prevents the flow of fluid through such pathways, and hence avoids allows better tissue regrowth, reducing any inhibition of tissue regrowth that may occur due to flow of fluid.

In addition to the first sealing surface provided around at least one of the hooks, the plurality of loops may be configured to provide a second sealing surface capturing each of the entry sites of each of the hooks. The second sealing surface may be formed of a loop capturing each of the hooks, or may be formed from portions of line and/or loops which in combination form sealing surface surround an outer perimeter around each of the hooks. The second sealing surface may be analogous to an O-ring surrounding an area substantially equal to a cross-sectional area of the anchor at the base of the anchor. The second sealing surface may therefore provide a sealing surface which captures each of the entry sites of the hooks.

The provision of two sealing surfaces (i.e. the one or more first sealing surfaces and the second sealing surface) may impede and/or prevent a flow of fluid through the entry sites of the hooks when the anchor is implanted in body tissue. This may in turn encourage tissue ingrowth around the anchor and thus improve the tensile strength of the anchor when implanted in body tissue.

The second surface may additionally provide a failsafe sealing surface, in the event that any one of the first sealing surfaces is not formed when the anchor is implanted in the body tissue. This may improve the reliability of the anchor in combination with a line in impeding and/or preventing high velocity jets and/or a flow of fluid flowing through the entry sites.

The knotting configuration may comprise a second knot. The second knot may be located at a distal end of the knotting configuration. The second knot may be formed at the base of the anchor. The second knot may form part of the second sealing surface. The second knot may comprise at least one of the plurality of loops.

The number of hooks may be a first hook and a second hook, and thus at least two of the hooks may be encircled by at least one loop of the plurality of loops. Thus the at least one hook may be two hooks, which may be the first and the second hooks. The knotting configuration may comprise a first knot at the base of the anchor body. The knotting configuration may comprise at least one loop around a first hook. The knotting configuration may comprise at least one loop around the second hook. The at least one loop around the first hook and the at least one loop around the second hook may be the at least one loop wrapped around each of the at least two hooks. The knotting configuration may comprise a second knot adjacent the loops around the first and second hooks, wherein the second knot is separated from the first knot by the loops around the first and second hooks.

The loops around the first and second hooks may be considered as each providing the first sealing surface. The loops around the first and second hooks, in combination with the second knot, may be considered as providing the second sealing surface.

The line may be formed of a biocompatible material. The biocompatible material may be ePTFE. The line may be a Gore-Tex^{®} ePTFE line. The line may be a ZEUS AEOS^{®} ePTFE line. The biocompatible material may facilitate tissue growth around the knotting configuration. The line may have a diameter of 0.1 - 0.6mm. The line may have a diameter greater than 0.4mm, or greater than 0.5mm.

The line may be configured to connect the anchor to a second anchor. The anchor may be a leaflet anchor, and the second anchor may be a papillary anchor.

The hooks may be formed with openings along their lengths. The openings preferably do not accommodate any part of the knotting configuration. The openings preferably do not accommodate any part of the line.

By adding openings in the anchor hooks a larger width hook can be used thereby increasing the holding strength while still allowing significant deformation between the folded and unfolded position without any plastic deformation. The increased surface area of the larger width hook also aids in spreading the distribution of forces. The openings may also enhance healing by allowing tissue to growing in between the slits, making a more reliable connection between the anchor and the tissue over time, rather than the tissue forming a "sock" that may be pulled out more easily, as would be the case with a solid hook.

In some examples the openings in the hooks include multiple holes (such as multiple holes of with a diameter of about 0.2-0.4mm), with these openings connected with a suture, wherein a single length of suture passes through several of the multiple holes, or all of the multiple holes. The suture is a distinct length of suture and/or line to the line in combination with the anchor. The suture may be knotted at each hole. The suture may for example be a Dyneema suture (or other similar suture, such as Dacron). Elastic materials such as nitinol can be prone to fatigue fracturing during high cyclic loads, including the cyclic loads that will arise from a beating heart. By the use of a suture through multiple holes it is possible to add a failsafe to the anchor hooks. If the hooks of the anchor break then the anchor is still kept together by the suture, which reduces risk for embolism while also providing extra time for ingrowth of tissue. Thus, even if an anchor breaks at an early stage then it will not embolise, and it will still be able to hold some force, as the expanded anchor will be too large to be pulled through its entry hole even if one or more hooks suffer a fracture. The use of a suture in this way will also make more "openings" for tissue to grow through. The multiple holes may be circular holes made in addition to other openings in the hooks, such as being made in addition to slits as discussed below.

As an alternative to the use of a suture threaded through the openings the anchor may include an overmolding, which may be provided about the entire anchor excluding the sharp tips of the hooks could be possible. A suitable material for such an overmolding is ePTFE. Another alternative is to use a woven fabric pouch that encloses the anchor. Both of these solutions would keep the anchor from embolising if there is a fracture in the anchor. The use of ePTFE also gives the added benefit of tissue ingrowth.

The anchor may be formed from a tube that is cut to provide tines extending from one end of the tube, with these tines then being curved and heat set to form the hooks. The end of the tube from which the hooks extend may be the anchor body. Openings can be cut into the tines before or after they are curved, but typically before in order that there is only one cutting stage. An added benefit of the use of openings in relation to this construction is that small diameter tubing becomes more pliable with an opening in the centre, since the arc of the tube is divided into two smaller arcs. As a result a wider section of a narrow tube can be safely utilized for making the tines which again gives additional strength. As a result of the increased holding force and increased pliability the anchor hooks are subjected to less fatigue load which in turn makes the implant last longer.

The openings may be formed as a series of holes, or as slits extending along the length of the tines to thereby extend along the curves of the hooks. A benefit of the use of slits is that each hook consists of two "legs" meaning that a fracture in one of the "legs" does not mean it will embolise, and the anchor will still be held in place by the other leg. At the same time the new "V" shape leg will highly likely grow into tissue more effectively than a straight "broken" hook without any slit or other openings, further reducing the danger of embolism.

The openings may include several smaller slits in line or have different types of pattern (zig-zag, barbed or wave pattern are examples). Along the length of the hooks, small holes with different patterns may be made, either instead of slits or in addition to slits. This can provide additional holding force, when tissue grows through the holes. It can also allow for a suture to be threaded through the hooks for added safety in the event of a fracture as discussed above. The slits may also be extended beyond the ends of the hooks where they join into the base of the anchor, which may be a tube shaped part as discussed above, thereby making the base more flexible as well. In some examples the slits may be cut as a single laser track. Circular openings can be added to the ends of such a cut to prevent high strain points.

In one example the anchor is cut from tubing made of an elastic metal, such as nitinol. Laser cutting may be used. This can involve cutting tines as discussed above, which can be heat set into curves. The anchor may be heat treated and/or electropolished. Chamfered edges may be introduced to the anchor on certain parts before the anchor is electropolished. The openings could contain a barbed or wave profile along edges of the openings, e.g. along edges of slits. Where slits are used the slotted hooks can be heat set in a configuration where they have increased distance when deployed. A barbed profile can then be concealed when the hooks are straight (barbs are facing towards one another). With this example, when the anchor comes to a non-constrained configuration then the slits move apart and the barb profile is engaged.

The anchor may be a leaflet anchor for use in the heart. The line may be an artificial chordae line. The body tissue may be a mitral leaflet. The anchor in combination with the line may therefore be a cardiac surgical device comprising the anchor and line as a leaflet anchor and artificial chordae line. The overall function of the leaflet anchor in combination with the artificial chordae line may be similar to the leaflet anchor in combination with a line of WO2016/042022. However, it will be appreciated that the features and structure of the anchor in combination with a line herein provides a departure from the prior art, adding benefits not found with the arrangement of the line proposed in WO2016/042022.

In various aspects the invention extends to the use of the anchors in combination with lines described above with catheter devices, and in particular to the use of those devices during a procedure for implanting an artificial chordae line into the heart. Further, the invention extends to the manufacture of the anchors in combination with lines described above, including various method steps such as laser cutting from tubes. For any of the anchors, or other laser cut parts discussed herein chamfered edges may be introduced before the laser cut part (e.g. anchor) is electropolished. Alternatively, the chamfered edges may be introduced by electropolish. The features of the second aspect and other optional features discussed above may be combined with the other aspects discussed below, with the anchors of those other aspects hence having a knotting configuration joining the anchor to a line in accordance with the second aspect.

Viewed from a third aspect there is disclosed a catheter device for repair of the heart by implanting an artificial chordae line, the catheter device comprising: a leaflet anchor for placement in a leaflet of a heart valve, wherein the leaflet anchor is arranged to be coupled to the artificial chordae line; and a leaflet anchor deployment mechanism for deploying the leaflet anchor to attach it to the leaflet of the heart, wherein the leaflet anchor deployment mechanism comprises a mechanical gripper device for grasping the leaflet of the heart valve, wherein the gripper device comprises a leaflet anchor tube for housing the leaflet anchor in a folded configuration; the gripper device and leaflet anchor being arranged such that when, in use, the gripper device grasps the leaflet, the leaflet anchor can be pushed out of the leaflet anchor tube to pierce the leaflet and form the leaflet anchor into an unfolded configuration so that hooked formations of the leaflet anchor can, in use, secure the leaflet anchor in the leaflet; wherein the mechanical gripper device includes a first gripper arm rotatably coupled to a main body of the catheter device so that the first gripper arm can rotate relative to the catheter device to move an outer end of the first gripper arm away from the main body of the catheter device and a second gripper arm rotatably and/or slideably coupled to the main body of the catheter device so that the second gripper arm can rotate and/or slide relative to the main body of the catheter device to move an outer end of the second gripper arm away from the main body of the catheter device; and wherein the first and second gripper arms are arranged so that they can move to come into contact with one another at a point spaced apart from the main body of the catheter device.

This arrangement can provide various advantages. For example, in the arrangement where the second gripper arm is rotatably coupled to the main body of the catheter device and/or where the second gripper arm can react to a sufficiently high force from the first gripper arm, then the use of two gripper arms allows for the leaflet to be gripped between the two arms at a point spaced apart from the main body, rather than only enabling the leaflet to be gripped between a single gripper arm and the main body, which is the arrangement described in WO2016/042022. The use of two gripper arms in this way can additionally or alternatively help stabilise a flailed leaflet, which is a leaflet segment without functioning chorda, that may flail into the atrium and be hard to catch with prior art devices. For example, the leaflet tends to move upwards which can make it difficult to catch the leaflet using a single gripper arm alone. Thus, in this regard, the second gripper arm may be considered as being a 'leaflet motion suppressor', as it may help to stabilise the flailing motion of the leaflet during a cardiac cycle. The use of a second gripper arm may also allow for a more horizontal gripping/contact surface (i.e. more perpendicular to the main body of the catheter device), which is beneficial both in terms of constraints on orientation of the main body, which is typically inserted from above the leaflet, and also has further advantages in relation to example embodiments in which the implantation of the leaflet anchor is carried out using the same device that implants a papillary anchor. In particular, the use of two gripper arms with a more perpendicular gripping location can facilitate the use of a device for performing the procedure of implanting both a leaflet anchor and a papillary anchor whilst the device remains in one place. It will be appreciated that the gripper arms may not necessarily be rigid structures, but may be flexible as required to achieve their desired operation.

In some examples, the use of two gripper arms allows for motion of the leaflet to be restrained between the two gripper arms at a point spaced apart from the main body. Thus, at (and near) the point(s) where the first gripper arm and the second gripper arm can contact one another then when the leaflet is present they will engage with the leaflet and restrict its movement. The leaflet tends to move upwards which can make it difficult to catch the leaflet using a single gripper arm alone. Thus, in this regard, the second gripper arm may be considered as being a 'leaflet motion suppressor', as it may help to stabilise the flailing motion of the leaflet during a cardiac cycle. Thus in this implementation, the second gripper arm may be slidably moved away from the main body of the catheter device to contact the top of the leaflet. The catheter device may be moved downwards such that the first gripper arm may then be rotatably moved away from the main body of the catheter device without contacting the leaflet or the second gripper arm, before being rotated back towards it. As the first gripper arm rotates back into the main body it then contacts the second gripper arm, which may for example be a flexible arm, restraining the leaflet between the two. The contact made by the first gripper arm against the second gripper arm may in this case be a slidable contact, allowing the first gripper arm to rotate back towards the main body whilst maintaining restraint of the leaflet. The first gripper arm then grasps the leaflet between itself and the main body of the catheter device. Hence as the first gripper arm is withdrawn back to grip the leaflet between the first gripper arm and the main body, as similarly described for the single gripper arm in WO2016/042022, the second gripper arm restrains the leaflet and prevents the leaflet from slipping out and thus the presence of the second gripper arm ensures the grasping of the leaflet in the first gripper arm. The use of a second gripper arm will also allow for a more horizontal gripping surface (i.e. more perpendicular to the main body of the catheter device), which is beneficial both in terms of constraints on orientation of the main body, which is typically inserted from above the leaflet, and also has further advantages in relation to example embodiments in which the implantation of the leaflet anchor is carried out using the same device that implants a papillary anchor. In particular, the use of two gripper arms with a more perpendicular contact location can facilitate the use of a device for performing the procedure of implanting both a leaflet anchor and a papillary anchor whilst the device remains in one place. It will be appreciated that the gripper arms may not necessarily be rigid structures, but may be flexible as required to achieve their desired operation.

The improved design may also allow large parts of the device to be produced from an elastic metal such as nitinol or stainless steel, and this in turn can allow for a production method that is reproducible and inexpensive. Alternatively, large parts of the device may be produced from a composite material, with choice parts formed from an elastic material such as nitinol or stainless steel as appropriate. The composite materials may comprise, for example, glass reinforced PEEK or carbon reinforced PEEK (CRF PEEK). Composite materials may have the advantage of improved imaging from ultrasound to allow monitoring during any procedure the catheter device is used for. Whilst composite materials may be not be as visible in x-ray imaging, radioactive markers or opaque contrast markers may be strategically located on the device to provide for such imaging. Composite materials may also be used for injection moulding of the components of the catheter device as required.

It will be appreciated that the leaflet anchor deployment mechanism of this aspect, as well as providing its own advantages, may also combine synergistically with the catheter devices of the aspects described below. Thus, it may be used to deploy the leaflet anchor in the device of the fourth aspect, for example with the leaflet anchor deployment mechanism placed in the proximal part of the two-part housing section. Alternatively or additionally it may be combined with the use of an ejector unit as disclosed in relation to the fifth aspect.

Capturing a leaflet with flail can be challenging, as it can move both "up" and "down" during a heartbeat. The gripper device of this aspect is equipped with an additional gripper arm to address this issue. The two gripper arms can both move relative to the main body of the catheter device. In some examples, the first gripper arm acts to enclose the second gripper arm, such that the first gripper arm must be rotated by a certain amount away from the main body of the catheter device before the second gripper arm can be freely rotated and/or slid within its entire range of movement. It may be that the second gripper arm can only be moved relative to the main body of the catheter device once the first gripper arm is opened to a certain degree.

The leaflet anchor tube may be housed within either the first gripper arm or the second gripper arm. The leaflet anchor is deployed by pushing it out of an opening at the end of the leaflet anchor tube, which is at the end of the respective gripper arm. In the example embodiments the leaflet anchor tube is within the first gripper arm, which may also enclose the second gripper arm as discussed above.

The two gripper arms may be operated individually to allow for independent movement. Alternatively, they may be linked in order that they move simultaneously similar to a "tweezers" mechanism. The use of two gripper arms can allow the upper gripper arm, which may be the second gripper arm, to make a "roof" for the leaflet, reducing the movement, and making the grasping easier especially when the leaflet is a complete flail. Another benefit is that the grasping action is more horizontal rather than vertical, i.e. more perpendicular to the main body of the catheter device than parallel to it.

In one example the first gripper arm may be arranged to be opened by rotation away from the main body, through 45 degrees or more, and preferably to an obtuse angle. The second gripper arm may be arranged to be enclosed by the first gripper arm when the first gripper arm is closed, and may be able to swing and/or slide outward from within the main body of the catheter device once the first gripper arm is open. Where the second gripper arm rotates then it may rotate with an opposite direction of rotation to the first gripper arm and may be arranged so that the rotation brings the end of the second gripper arm into a path of movement of the end of the first gripper arm. A centre of rotation for the first gripper arm may be spaced apart along the length of the main body of the catheter device compared to a centre of rotation of the second gripper arm. It should be noted that the centre of rotation may not be fixed as there may be some deformation of the device during the rotation process, for example the first gripper arm may rotate by bending of a flexible section of material, which can lead to movement in the centre of rotation depending on the degree of bending. In cases where the second gripper arm slides then it may slide to move its end outward from the main body of the catheter device and into the path of movement of the end of the first gripper arm.

The gripper arm(s) may be moved by pulling one or more wire(s), which may be connected to lever arms joined to the gripper arm(s). With the second gripper arm open (i,e, rotated or slid outward from the main body) with its end spaced apart from the main body, for example with the second gripper arm extending at an angle of between 45-90 degrees from the main body, then the first gripper arm may be rotated in the closing direction toward the end of the second gripper arm, such that the first gripper arm moves to contact part of the second gripper arm.

In some examples the gripper device may capture the leaflet, and/or restrain its movement, by engagement (contact) of the two gripper arms, which may be done by rotation of one or both arms. The second gripper arm may also be individually moved during the gripping action. The two gripper arms may move in order to engage a gripping surface of the second gripping arm with a gripping surface of the first gripper arm.

The gripper device may capture the leaflet by first restraining it between a contact point between the second gripper arm and the first gripper arm. The first gripper arm may then be rotated closed, i.e. towards the main body of the catheter device, such that the restrained leaflet is successfully grasped by the first gripper arm. The second gripper arm may remain fixedly in place during motion of the first gripper arm.

For the gripper arm that houses the leaflet anchor tube, which may be the first gripper arm, the gripping surface may be a gripping platform located around the opening of the leaflet anchor tube. Whilst the leaflet is gripped between the two gripper arms or a gripper arm and the main body of the catheter device, the leaflet anchor is placed, for example using any technique discussed above and then the gripping device is opened, for example by rotation of the first gripper arm away from the second gripper arm and/or the main body of the catheter device. Where the device of this aspect is combined with the device of the fifth aspect and hence an ejector unit is present, then the connection of the leaflet may be tested after the gripping device is opened to ensure proper placement of the leaflet anchor in the leaflet prior to release of the leaflet anchor from the ejector unit.

The second gripping arm may be actuated with two wires, allowing the physician to move it in two rotating or sliding directions to aid in the grasping process.

The second gripper arm, i.e. the leaflet motion suppressor, may be a flexible member and/or may comprise a wire. The wire may be formed of an elastic material such that it may be contained, housed, stored and/or sheathed within a lumen of the main body of the catheter device when not in use. The elastic material may be nitinol or stainless steel, for example. This advantageously gives a user of the device the decision of whether or not the use of the second gripper arm is desired during placement of the leaflet anchor.

The leaflet motion suppressor comprising an elastic wire may be in an elastically deformed state when stored within the lumen. However, when the leaflet motion suppressor is moved away from the main body of the catheter device the leaflet motion suppressor may return to an undeformed state. The leaflet motion suppressor may be slid out of the lumen to move its end away from the main body of the catheter device.

The leaflet motion suppressor may comprise a number of shapes and/or arrangements capable of suppressing flail of the leaflet, to ensure engagement of the leaflet between the first gripper arm and the second gripper arm, when the leaflet motion suppressor is in its undeformed state.

In one example the second gripper arm may be a looped nitinol wire that is pushed out of the proximal end of the device, by pushing the two proximal wire ends toward the distal end of the device, a looped wire extends out of the proximal end of the gripper housing. The loop in the wire may advantageously stabilise the leaflet motion suppressor as it engages the leaflet. The loop of wire may encompass a large surface area which assists with engagement of the leaflet.

The loop of wire may also prevent the leaflet motion suppressor from being withdrawn completely into the catheter device. That is, the loop of wire may engage with a feature of the lumen such as a pin, such that a distal end of the wire is always outside and/or flush with a main body of the catheter device. Thus for the leaflet motion suppressor comprising a wire, a portion of the wire and/or an end of the wire may be located outside of/flush to an outer surface of the proximal part of the main body of the catheter device.

In another example, the second gripper arm may be an open-ended and/or loose wire, i.e. a wire wherein at least one of the ends is located outside the main body of the catheter device when in the undeformed state. The wire being open-ended and not forming a loop may help prevent entanglement of the leaflet in the leaflet motion suppressor. In this arrangement, the leaflet motion suppressor may comprise a number of bends and/or curves parallel to the plane of the leaflet, which advantageously increases the surface area of engagement between the leaflet motion suppressor and the leaflet. To prevent the leaflet motion suppressor from being completely withdrawn into the catheter device, the wire may comprise a wire stopper at its end, the wire stopper being a feature such that the wire stopper is wider and/or larger than the lumen.

When the leaflet motion suppressor comprises a wire with at least one end of the wire configured to be outside the main body of the catheter device, the leaflet motion suppressor may undesirably pierce and/or damage the leaflet or surrounding tissue as the second gripper arm is slid out of and/or moved away from the main body of the catheter device. With the aim of preventing this disadvantageous effect, the bends and/or curves of the wire may be formed such that the end of the wire is configured to point away from the leaflet. For example, the end of the wire may curve away from a surface of the leaflet, or may point in a direction opposite to that which the second gripper arm is to be moved. Additionally and/or alternatively, the end of the wire may comprise a soft tip to decrease the chance of puncturing the surrounding tissue.

In one example, the undeformed shape of the leaflet may comprise a spiral, the spiral forming a large engagement surface between the leaflet and the second gripper arm. The spiral may also be formed such that the end of the wire located outside the main body of the catheter device is at the centre of the spiral. Advantageously, this decreases the likelihood that the end of the wire pierces and/or damages the surrounding tissue as the end of the wire is less exposed.

The lumen in which the leaflet motion suppressor is stored may comprise a channel, path and/or conduit running along a length of the catheter device parallel to a main axis of the catheter. However, where the lumen meets the mechanical gripper device the lumen may angle towards an outer surface of the main body of the catheter device such that the leaflet motion suppressor may slide out of the lumen to engage the leaflet. The lumen may be angled such that the leaflet motion suppressor leaves the lumen perpendicular to a surface of the main body of the catheter device.

The wire component of the leaflet motion suppressor may be an off-the-shelf wire, such as a guide wire, readily available for use in cardiac interventions. Accordingly, an operator of the catheter device can then choose a wire that they find appropriate for suppressing motion of the leaflet during an operation. In other words, different wires of an identical predefined size may be implemented with different stiffness and/or tip structure (i.e. bends, curves and/or loops) as desired. For example, if a first wire did not function as desired, a second wire having similar or different characteristics may be used. As such, the leaflet motion suppressor may not be stored within the lumen of the catheter device permanently, but may be selected from a storage device and inserted into a port of the catheter device during a particularly challenging procedure. This approach has the further advantage that it may use wire components for which regulatory approval has already been granted, and/or wire components that the user is familiar with from other types of cardiac interventions.

The first gripper arm may be actuated with a single wire or with multiple wires. Advantages can be obtained if a hinge mechanism for the first gripper arm is formed integrally with the material of the main body and rotates away from the main body by elastic deformation of that material. The first gripper arm as well as the hinge mechanism may be formed integrally with the material of the main body. Alternatively, the first gripper arm may include a separately formed arm section, such as a milled piece or a laser cut piece, with the separate arm section being attached to a hinge mechanism of the main body, for example by gluing or welding.

In a slightly different arrangement the second gripper arm may be attached to the base (somewhere close to the rotational "axis") of the first gripper arm. This second gripper arm may be an elastic material such as nitinol. In a default configuration the second gripper arm may follow the gripping surface (inner surface) of the first gripper arm with a slight pressure towards the gripping surface of the first gripper arm, with the pressure being induced by tension in the material of the second gripper arm. The arrangement can be compared to a "reversed" tweezer, where a force is needed to open it. The reversed tweezer follows the movement of the first gripper arm unless there is a force that pulls it open, the force could for example be in form of a pull wire, or wedge placed in between the first and second gripper arm.

In some examples, the main body of the catheter device may be formed from an elastic metal such as nitinol with a hinge being provided by an elastic joint formed in the elastic metal. In that case a single wire can be used to elastically deform the first gripper arm by bending an elastic joint with the main body to rotate the end of the first gripper arm away from the main body, with the first gripper arm returning elastically to its at rest position once no force is applied to the wire. An advantage of this is that the elastic force of the first gripper arm can hold it in place against the second gripper arm when the force is released from the wire, without the need for a separate wire to be pulled to keep the grip on the leaflet secure. A second wire may however be implemented as a backup if it may be needed.

In other examples, the main body of the catheter device may be formed from a composite material, such as carbon or glass reinforced PEEK. The first gripper arm may then be joined to the main body of the catheter device using a pin joint, the pin forming the axis of rotation of the first gripper arm. Similarly, when the second gripper arm comprises a sheet of elastic metal, the rotatable element of the arm may be formed by another pin joint located on the surface of the main body of the catheter device. The pin joint mentioned herein may be a revolute joint or a hinge joint, i.e. comprising intermeshing features with a pin or cylindrical member joining said members, the pin forming the axis of rotation for the joint. The motion of the second gripper arm may then be controlled by one or more pullwires, as described above. When the second gripper arm comprises a single wire as described above, the lumen may be formed through the composite material to allow passage of the leaflet motion suppressor in and out of the catheter device.

Alternatively or in addition the first gripper arm can be heat set in a "more than closed" configuration. This would allow the first gripper arm to grasp tissue towards the main body of the device as well as towards the second gripper arm.

To form both the first gripper arm and the hinge integrally with the main body of the catheter then the main body of the catheter may comprise an outer tube, with the first gripper arm being formed as an articulated section of the outer tube. Several forms of slits and/or patterns can be formed in the tubing in order to provide a weakened hinge section allowing for bending without plastic deformation of the first gripper arm.

In alternative arrangements a hinged gripper arm may be used. In that case the first gripper arm may be milled, actuation in that case could be done with a spring for closing, and wire for opening, or vice versa, or with two wires (one for opening and one for closing). A pulley cut in the device can be used to redirect the pulling force from the pull wire.

One or both gripping surface(s) may be arranged to hold the leaflet with friction. For example the gripping surface(s) may use a material with a high coefficient of friction and/or the gripping surface(s) may have a texture or surface profile for increasing friction, such as a ridged or saw-toothed profile. The end of the leaflet anchor tube typically opens into one of the gripping surfaces. The leaflet anchor tube may take the form of a generally cylindrical channel sized to be slightly larger than the leaflet anchor in its folded configuration.

The leaflet anchor may be formed from an elastic material and to be arranged so that it assumes the unfolded configuration when no force is applied, and to be able to deform elastically into the folded configuration, for example when constrained within the leaflet anchor tube. Further possible features of the leaflet anchor are discussed at various points below.

It is advantageous if the leaflet anchor can be placed into the leaflet from beneath, i.e. from the side where the papillary muscle is located. To facilitate the preferred placement of the leaflet anchor from beneath, the catheter device may be arranged so that the open end of the leaflet anchor tube is at a proximal end of the gripper device (the 'upper' end when in the heart in the above defined orientation) and the leaflet anchor can be pushed out of the channel moving from the distal end of the catheter device toward the proximal end. Thus, the end of the first gripper arm may also have the end of the leaflet anchor tube, and this may be arranged to direct the leaflet anchor in a direction extending toward the proximal end of the catheter device. In some embodiments the catheter device includes a U-shaped rod for deployment of the leaflet anchor, as discussed further below.

In some examples the second gripper arm can be cut out of the main body of the catheter device in a similar way to as the first gripper arm, for example cut from a piece of the main body at an opposite side of the main body to the first gripper arm. This second gripper arm could have cut features in its base, allowing for a tight bend being pulled out of the device, and may also be heat formed for increased stiffness.

In examples using a mechanical hinge for the first gripper arm the catheter device main body could be made of an elastic metal such as nitinol while the first gripper arm itself is milled from stainless steel otherwise formed separately. Alternatively, the main body may be milled with the gripper arm cut from elastic metal, or the entire device could be milled or made by additive manufacturing.

The leaflet anchor tube can be heat treated with a flattened section on its inner end that extends past the first gripper arm's "centre" of rotation. This can act as a lever for pulling the first gripper arm open.

The second gripper arm may be cut from sheet metal, such as nitinol, and placed within the main body of the catheter device in an elastically deformed state. This deformation may be purely to allow the arm to take a smaller profile for insertion into the main body, so that it will expand into a non-deformed state once it is within the main body. Alternatively, some elastic deformation may remain once the second gripper arm is within the main body, for example, so that it will retain itself in place via elastic forces and/or so that it may automatically deploy by unfolding elastically when the first gripper arm is opened. The second gripper arm may be formed with heat setting with for example a light curve or a convex curve to improve stiffness and or provide a gripping surface. Wave or barbed edges may be provided in order to enhance the gripping strength of the gripping device. In addition, or alternatively slits can be placed on the surface of the second gripper arm to provide different flexing properties. In some examples a hinge mechanism for the second gripper arm is formed in the main device by the use of two holes, with pins formed in the second gripper arm that fit into the holes. This may be assembled by inserting the second gripper arm with elastic deformation as discussed above, and by allowing the second gripper arm to fully or partially unfold into a position where the pins engage with the holes to make the hinge. Wires can be attached to the second gripper arm to move it up and down, or it could be spring loaded one way, and pulled the other way.

When the two armed gripper of this aspect is combined with the fourth aspect and its flexible joint, then in one example the two-part housing section of the fourth aspect is made from a single tubing section cut to a required shape, with the first gripper arm being provided in the proximal part of the two-part housing section, which forms the main body of the catheter device, and with the first gripper arm advantageously being cut from the same tubing section. In this way it becomes possible to create many features of the catheter device from a single tubing section, such as from laser cut nitinol. Alternatively the two-part housing section of the fourth aspect is made from two parts coupled with a hinge, as discussed above, and in this case the catheter device may be formed from a combination of materials, perhaps including composite materials.

Viewed from a fourth aspect there is disclosed a catheter device for implanting a leaflet anchor and a papillary anchor into the heart as part of a procedure for implanting an artificial chordae line that extends between the leaflet anchor and the papillary anchor, the catheter device comprising:
a two-part housing section extending from a distal end of the catheter device along the length of the catheter device toward the proximal end of the catheter device, the two-part housing section being arranged to be placed between the papillary muscle and a leaflet of the heart during use of the catheter device, and the two-part housing section comprising a distal part at the distal end of the catheter device and a proximal part located on the proximal side of the distal part;
a leaflet anchor deployment mechanism at the proximal part of the housing section for deploying a leaflet anchor for attachment to the leaflet of the heart;
a papillary anchor deployment mechanism at the distal part of the housing section for deployment of a papillary anchor for attachment to the papillary muscle, wherein the papillary anchor deployment mechanism is arranged for deployment of the papillary anchor by moving it outward in the distal direction relative to the distal part; and
a flexible joint located between the proximal part and the distal part of the two-part housing section, wherein the flexible joint allows a centreline of the distal part to be angled relative to a centreline of the proximal part.

The device of this aspect provides a new method to insert the papillary anchor that may allow the physician to implant the leaflet and papillary anchors without the need to move the device after first placing the leaflet anchor, or after locating the device ready to place the leaflet anchor/grasp the leaflet (with the papillary anchor being placed first in the latter situation). In contrast to the device described in WO2016/042022 the catheter does not necessarily need to be moved to a different orientation or position within the heart before the papillary anchor is placed. Instead the flexible joint may allow for the distal part to be angled toward the papillary muscle area while the remainder of the catheter device is not moving. The flexibility of the joint, can also allow for the distal end of the distal part to push more evenly against the papillary muscle, i.e. to ensure that it presses against the body tissue more evenly across the whole cross-section of the distal end. In turn this ensures effective implantation of the papillary anchor, since it can engage with the body tissue around the whole cross-section.

This device hence reduces the risk of entanglement as well as minimising the time needed for the implanting procedure. In WO2016/042022 a method to place the anchor is described but the design of the papillary anchor deployment mechanism and its housing needs greater care to ensure that all of the anchor pins were well engaged with the body tissue. It should be noted here that in this document the term "pins" is used interchangeably with the term "hooks" and the same elements of the anchor is described in each case.

Optionally, the flexible joint may also be extendable. Thus, there may be a flexible and extendable joint between the proximal part and the distal part of the two-part housing section. The flexible and extendable joint may allow the distal part to be moved away from the proximal part via extension of the joint to thereby extend the distal end of the catheter device further into the heart. In this way the device can be extended to move the distal part of the housing section along with the papillary anchor in a direction toward the papillary muscle area while the remainder of the catheter device is not moving. The resilience of the flexible (and extendable) joint can act to avoid excessive force on the body tissue, reducing the risk of trauma during implantation, as well as aiding in ensuring an even pressing force with the extending and flexing mechanisms working in combination.

The papillary anchor may consist of a number of pins that are arranged to form hooks in the body tissue as the anchor moves out of the distal part of the housing section into a deployed configuration. In some examples a papillary anchor of similar design to that of WO2016/042022 could be used. In other examples the papillary anchor may have further features as discussed below, such as slits along the pins. The proposed device of the fourth aspect might also be used with other types of anchors that need to be placed at a distance, such as a screw anchor or a barbed anchor.

As explained above, by adding a flexible joint between the two parts of the housing section a more reliable deployment and lower chance of entanglement can be achieved. The flexibility of the joint also helps the device travel through bends in the catheter as it is split into two shorter straight parts that can flex relative to one another, rather than being one long rigid section. The flexible joint allows a centreline of the distal part to be angled relative to a centreline of the proximal part, and these centrelines may be aligned with a centreline of the catheter when the device is at rest. It will be appreciated that the device will have a prismatic form, typically cylindrical, and the centrelines may hence be along the centre of the cross-section of the prism. During use of the device the centreline of the proximal part of the housing section may remain aligned with a centreline of adjacent parts of the catheter that support the housing section, whereas the centreline of the distal part may be angled differently.

The optional feature of an extendable joint also allows the distal part to be moved away from the proximal part to thereby extend the distal end of the catheter device further into the body/heart, and thus it has a telescopic effect that changes the overall length of the two-part housing section. Where a flexible and extendable joint is used this may have two separate mechanisms to provide the required flexibility and extendibility. Thus, there may be a mechanism arranged for bending between the two parts, and a separate mechanism for extension via some form of telescopic effect. The telescopic effect might in this case be provided by a sliding sleeve arrangement, by foldable or hinged structures, and/or by elastically collapsible structures. In other examples, including the example embodiment illustrated herein, the flexible and extendable joint may have a single "flextendable" part providing both the flexing and extending functions. This may for example be a foldable and/or elastically collapsible structure, such as a bellows arrangement (as with flextendable drinking straws) or a structure with one or more collapsible coil and/or wave shapes, such as coil springs or a set of parallel meandering paths.

The two-part housing section may be formed from two tubular sections in any suitable material, i.e. a medically appropriate material. Stainless steel or nitinol may be used. In the alternative, composite materials such as carbon-fibre or glass-fibre reinforced PEEK may be used. The catheter device may be formed via a combination of such materials with the materials for different parts of the device being selected dependent on the required characteristics of those parts. A material that allows Ultrasound to pass through and at the same time have sufficient strength is preferred, Carbon reinforced PEEK meets these demands well, and would also allow Injection moulding of the components which lowers manufacturing cost. Fibre reinforced plastic are normally not visible on X-ray, so strategically placed radiopaque markers in all components may be used to determine device component(s) position and orientation on X-ray relative to each other, as complementary information to ultrasound imaging.

In some example embodiments a flextendable element is formed by providing collapsible forms into the walls of a tube made of a flexible and elastic material, such as nitinol or another shape memory metal. Laser cutting may be used to provide the required forms. The extendable and flexible joint can be cut with any suitable pattern to achieve the required functionality. For example, it may be formed as a regular (e.g. helical) spring. The extendable and flexible joint may be cut with asymmetry to achieve desired flex patterns and asymmetric forces during contact of the distal end with the wall of the heart. A thin walled silicone element is a possible alternative to a tube cut into collapsible forms. For example, a thin walled silicone tube that can be stretched many times its original length. In that case the silicon tube part may be connected to the gripper section and papillary anchor section via suitable support brackets.

The flexible and extendable joint can be extended during the procedure for insertion of the papillary anchor, as discussed further below. It can also be extended independently or be under compressive-tension prior to insertion and then be released (making the device longer, pushing the heart wall).

It is also possible to use the flexible and extendable feature individually, i.e. not in direct conjunction with the placement of the leaflet anchor. Thus, the procedure could be split into two stages, one for attaching sutures to the leaflet, and one for placing the papillary anchor. When the steps are done individually there may be advantages from using a telescopic tube to provide all or a part of the extendable function, as the device can be made shorter with that approach.

The joint may have mechanical shielding internally and/or externally to prevent wires, chordaes or tissue from getting pinched. This may be in form of a flexible membrane that stretches with the extendable joint, for example a thin sleeve that sits outside the joint. The membrane may be a silicone membrane which is fixed onto the outside of the unit above and below the joint. For example it may be fixed with adhesive. Alternatively a flexible layer of silicone (or other flexible material) could be over moulded onto the flexible joint to reduce pinch risk during movement of the joint. Fabric covering techniques similar to what is done to cover oesophageal stents or stent grafts may be applied to the joint.

In some examples using a flexible and extendable joint the joint may be covered by a tube section that extends all the way to the distal end of the catheter device when the flexible and extendable joint is compressed. This may for example be a thin walled nitinol tube. This allows the extendable joint to be completely covered during its entire travelling length. The covering tube may reduce the amount of flex in the device, therefore a further flexible section may be added just above where the covering tube is attached to the device, for example by cutting a pattern. The covering tube may be welded or glued onto the device body.

The delivery handles used by the operator to control the device may be coupled in such a way that the artificial chordae line(s)are extended when the lower section of the device is extended, in order to hold the chordae in proper tension independent on how much the lower section of the device is extended. Additionally or alternatively, a constant tension device such as a constant force spring may be disposed in the delivery handles to achieve proper tensioning of the chordae and thus remove any slack in the chordae line(s). The removal of slack from the chordae by keeping the line(s) in tension may prevent entanglement of the chordae between itself and any other components in the device.

The flexible and extendable joint can be formed in a default extended, compressed or somewhere in-between "spring configuration", to allow different means for movement/functionality. It could also be heat set partly stretched, which can allow for reduced use of material.

The leaflet anchor and/or the leaflet anchor deployment mechanism may be similar to that of WO2016/042022. Alternatively or additionally the leaflet anchor and the leaflet anchor deployment mechanism may have features as discussed below.

The papillary anchor is housed within the distal part of the housing section before its deployment. The papillary anchor may have a similar cross-section as the distal part of the housing section. For example, both may have a tubular form when the anchor is held in the distal part. As noted above the anchor may have a folded and an unfolded configuration allowing pins of the anchor to form into hooks within the body tissue during deployment of the papillary anchor. The papillary anchor deployment mechanism may take a similar form to that of WO2016/042022, and/or it may have further or alternative features as discussed below.

In one example the papillary anchor deployment mechanism includes a first wire or rod for pushing the papillary anchor in the distal direction relative to the distal part of the two-part housing section. There may additionally be a second wire or rod for releasing the papillary anchor from the papillary anchor deployment mechanism in order to disengage the papillary anchor from the catheter device after it is implanted in the body tissue, i.e. the tissue of the papillary muscle and/or tissue adjacent to the papillary muscle.

The papillary anchor may have a chordae line attached to it, and may include a locking mechanism, such as a locking ring as in WO2016/042022 and as discussed below, the locking mechanism being for clamping the chordae line when no force is applied to the locking mechanism. The locking ring may be able to be elastically deformed to release the line from the locking mechanism for adjustment of the length of the chordae line. The papillary anchor deployment mechanism may include a locking ring holder for holding the locking ring in its elastically deformed position, with the papillary anchor deployment mechanism being arranged to selectively withdraw the locking ring holder from the locking ring so that the chordae line can be locked in place after deployment of the papillary anchor and after any required adjustment of the length of the chordae line. This locking ring holder may have a Z-shape as discussed below.

The flexible joint may include a hinge element, for example with the distal part of the two-part housing section coupled to the proximal part via a pivoting mechanism or via an elastically deformable element. For example, the two parts of the housing section may be composite or metal parts coupled together by the hinge element.

In some examples the flexible joint is controllable via one or more wires, such as nitinol or stainless steel wires. There may be a wire allowing for control of the angle of the flexible joint by pushing and/or pulling. There may be three wires that are distributed in a support section in the housing section and/or attached to the flexible joint, for example to achieve a complex movement, such as where the joint is also extendable. These wires may be arranged so that when one or more wires is pushed or pulled then this will control movement of the distal part of the housing section. For example they might change the angle or extension of a flexible and extendable joint. The three wires may be arranged to be used by pushing or releasing in order to extend the device to retrieve a placed papillary anchor while still holding the leaflet in the gripper. The wires may also be arranged to be used to angle the distal part to be more perpendicular to the heart wall, for a more optimal placement of the papillary anchor.

In some examples the hinge element is controllable via one or more hinge pullwires. The hinge pullwire(s) may be of the form of the one or more wires described above. The hinge pullwire(s) configured to control the hinge element may be arranged to sit inside and/or pass through the front of the catheter device (wherein 'front' refers to the side of the catheter device shaft where the leaflet anchor deployment mechanism may be located). The hinge pullwire(s) configured to control the hinge element may also be arranged such that they are radially offset from a central axis of the catheter device, i.e. such that they are proximate a wall of the catheter device rather than a central axis of the catheter device.

When the hinge pullwire(s) configured to control the hinge element are arranged as described above, the hinge pullwire(s) may act as a deflection wire, i.e. the hinge pullwire(s) may be configured such that when the distal part of the two-part housing section is angled relative to the proximal part of the two-part housing section through the use of the hinge pullwire(s), the hinge pullwire(s) may deflect a device shaft of the catheter device in the same direction that the hinge element of the flexible joint is angled. This may have the effect of increasing the force acting on the wall of the heart during deployment of the papillary anchor from the catheter device. The actuation of the hinge element and the deflection of the device shaft may be sequential or simultaneous during operation of the hinge pullwire. For example, during operation of the hinge pullwire the device shaft may deflect at the same time the hinge element bends, or during the operation of the pullwire the hinge element may bend first and the device shaft may deflect second. Additionally, when the hinge pullwire(s) is actuated the device shaft of the catheter device may be steered in a target direction. This beneficially assists in ensuring that the distal part of the two-part housing section is perpendicular to a target wall of the heart during papillary anchor deployment.

In some examples the flexible (and optionally extendable) joint is cut with laser from an elastic tube (for example a nitinol tube), that also acts as the structural component of the entire catheter device, such that the tube also forms the distal part and proximal part of the two-part housing section. Different types of patterns can be applied to the tube edge towards the tissue to achieve different friction and/or potential "hooking" to keep the device stable during implantation, one example is a wave pattern edge or a flange with increased surface towards the tissue. To avoid pinching of the new chordae a sheath to cover the suture inside the joint can be implemented, wherein the suture sheath can be retracted/opened once the placement of the anchor is confirmed.

An example of the use of the catheter device of the fourth aspect may include the following steps: (1) the device is first placed in near proximity to final placement; (2) the flexible joint is angled to move the distal part toward the papillary muscle and the wires/rods along with the papillary anchor within the distal part move with it, for example due to friction between the papillary anchor (or a papillary anchor push tube) and the internal surface of the distal part of the housing section; (3) the distal end of the distal part meets the body tissue, and as force is applied the counterforce from the body tissue eventually surpasses the forces holding the papillary anchor in place, at this point tissue is pushed flat below the base of the device giving a maximal chance of placing all pins correctly in tissue, and force can be applied to the anchor so that the ends of the pins then move beyond the distal end of the distal part to meet the body tissue, this may be done via additional force on the anchor from rods or wires, or advantageously it may be done through a pre-tension on the anchor that is held by friction with the distal part until the forces from the body tissue on the distal part changes the balance of forces with the friction sufficiently so that the papillary anchor ejects (similar to a paper stapler); (4) the papillary anchor pins fold out and form into the hook shape of the unconstrained papillary anchor to thereby engage with the body tissue, at which point the connection can be pull tested by operator, and/or visually confirmed on x-ray and/or ultrasound; (5) if the connection is not satisfactory, the papillary anchor can be pulled back into the device and re-placed to attempt an improved coupling of the anchor with the body tissue. The same device may also implant the leaflet anchor, which can be done after implantation of the papillary anchor, or optionally prior to implantation of the papillary anchor. During the implantation of the papillary anchor the leaflet anchor deployment mechanism may be used to grip the leaflet, with or without deployment of the leaflet anchor.

It will be understood that the operation of the catheter device of the fourth aspect to implant the papillary anchor may be compared to a paper stapler, since force on the device end (when being pushed) will drive the papillary anchor out of the end and into the material adjacent the end similar to a stapler. In a typical example, once the device is in position and the leaflet is secured (for example in a gripper as in WO2016/042022, or as discussed below) then the papillary anchor can be placed, if placement of papillary anchor is approved, the leaflet anchor can be placed, if not then the leaflet might be detached and papillary anchor retrieved to be placed again. The flexible joint in the centre of the device also improves movement through the catheter, especially through arcs, as it can more easily go through curves as two shorter components connected with a flexible joint.

In some examples the actuation of the leaflet anchor can be connected to the papillary anchor deployment, meaning that the leaflet and papillary anchor may be at least partly deployed at the same time. This can make the procedure easier and/or faster.

Viewed from a fifth aspect there is disclosed a catheter device for implanting a leaflet anchor during a procedure for implanting an artificial chordae line into the heart, the catheter device comprising: a leaflet anchor for attachment to the leaflet of the heart; and a leaflet anchor deployment mechanism for deploying the leaflet anchor; wherein the leaflet anchor deployment mechanism allows for retraction and repositioning of the leaflet anchor after deployment of the anchor into the leaflet via an ejector unit having a grasping device with a first configuration arranged to permit deployment of the leaflet anchor into the leaflet without disengagement of the leaflet anchor from the ejector unit, and a second configuration in which the leaflet anchor is reversibly released from the ejector unit; wherein in the first configuration the grasping device of the ejector unit grasps a proximal end of the leaflet anchor, whilst a distal end of the leaflet anchor is unimpeded by the grasping device to enable it to be implanted in the leaflet; and wherein in the second configuration the grasping device of the ejector unit is disengaged from the leaflet anchor.

The leaflet anchor may be retracted with a retraction tube/catheter, by pulling the chordae so the leaflet anchor folds inside the retraction tube. The retraction tube may be placed on top of a chordae only attached to the leaflet (with device removed) or a leaflet anchor placed in a poor location (partly engaged, free floating, entangled etc.). The retraction tube may be a deflectable shaft, with or without a flexible section on the tip (that allows the tip to find the leaflet anchor base, to allow retraction). Alternatively the retraction shaft may be a flexible tube that is arranged to engage with the base of the leaflet anchor. In either configuration a marker band in the tip is needed to confirm that the retraction tube is at the base of the leaflet anchor, prior to applying tension to the chordae, to prevent any unwanted damage to the implant or native tissue.

Another alternative to retract the leaflet anchor when it is free floating (not attached to anything) is to tension the chordae until the leaflet anchor can be folded inside the papillary anchor housing, either in the distal end or through an opening in the papillary anchor housing wall.

As will be seen from review of WO2016/042022, in this earlier proposal the leaflet anchor is pushed out once the gripper of the leaflet anchor deployment mechanism holds the leaflet and after being pushed out the leaflet anchor cannot be retrieved with the same mechanism. Whilst it is possible to retrieve the leaflet anchor with the device of WO2016/042022, there is only one relatively complex way described to do this, and it involves a separate retrieval catheter. With the catheter device of the fifth aspect, in order to give the physician additional control, an "ejector unit" is introduced that allows for the leaflet anchor deployment mechanism to deploy and also retrieve the leaflet anchor.

It will be appreciated that the features of the device of the fifth aspect may be combined with those of the fourth aspect, thereby achieving the advantage of each. Moreover, there is synergy in this combination since the ability to remove and replace the leaflet combines with the benefits of the ability to keep the catheter device in place at the leaflet whilst the papillary anchor is inserted via use of the flexible and optionally extendable joint. This allows for the surgeon maximum flexibility in terms of insertion of the two anchors and checking of the connections before any significant motion of the device is needed away from its position at the leaflet anchor. The device may also be moved from the leaflet anchor placement position to accommodate papillary anchoring position or the other way around.

The telescopic shaft that holds the device may be fitted with 4 pullwires, so that the distal tip can move in order to locate correct valve position for placing the anchor(s).

The leaflet anchor may be formed from a flexible material with a hooked shape in an unfolded configuration, and being able to deform elastically into a folded configuration, for example when constrained by the leaflet anchor deployment mechanism. The material of the leaflet anchor may be nitinol. The shape of the leaflet anchor may include hooks that are straightened out when the leaflet anchor is in the folded configuration. The hooked shape of the unfolded configuration may be a grapple hook shape, for example. The leaflet anchor may have a similar form to that of WO2016/042022 and/or may have features as described below. In example embodiments, the leaflet anchor and leaflet anchor deployment mechanism may be arranged such that the when the leaflet anchor is pushed out of the leaflet anchor deployment mechanism then this can drive the hooks though the leaflet whilst the hooks return elastically to the unfolded configuration, thereby securing the leaflet anchor in the leaflet.

In example devices the chordae sits inside a groove in the device, after the leaflet anchor is placed, and applying tension (shortening) of the chordae may be used in order to release the chordae from the groove it sits in. Removing slack in the system can reduce the chance of the chordae wrapping around the device, creating complication. An example of a device to reduce slack may be some sort of constant tension device, such as a constant force spring. The constant tension device may be disposed in a delivery handle of the device.

The ejector unit may be placed within the leaflet anchor deployment mechanism inboard of the leaflet anchor. With this arrangement, when the ejector unit and leaflet anchor are within the leaflet anchor deployment mechanism then the ejector unit holds the leaflet anchor with the grasping device in the first configuration. The leaflet anchor deployment mechanism can deploy the anchor to implant it in the leaflet. In example embodiments, the grasping device may be arranged to remain in the first configuration during this deployment, with the ejector unit being arranged so that it moves to the second configuration only after the leaflet anchor is implanted. With the leaflet anchor implanted the grasping device can be used to test the connection of the anchor to the leaflet, by a force being applied to the leaflet anchor from the ejector unit whilst the grasping device is in the first configuration. Another way to test the connection is to assess leaflet movement compared to the blood flow, with the leaflet attached to the leaflet anchor and thereby held to the catheter device, i.e. before the leaflet anchor is released. If the leaflet anchor is well-engaged then the movement of the leaflet will be more restricted than if it is not well-engaged. Subsequently, with the ejector unit moved into the second configuration, the grasping device of the ejector unit opens and at this point the physician may further test the connection of the anchor to the leaflet, for example via tension applied to the chordae line. If the physician is not satisfied (for example, if there is too much movement of the anchor and/or not enough resistance to force on the line) then the leaflet anchor can be retracted and placed in another location. If the grasping device did not change from the first configuration during the test then the latter procedure may be carried out by reversing the deployment of the ejector unit and leaflet anchor, for example by drawing those parts back into the leaflet anchor deployment mechanism. If the second configuration was used before it was determined that the connection of the anchor was not adequate then to retract the anchor the ejector unit is first moved back to the first configuration so that the grasping device reengages with the leaflet anchor, and then after that the deployment of the ejector unit and leaflet anchor is reversed, for example by drawing those parts back into the leaflet anchor deployment mechanism.

The use of the device of the fifth aspect reduces the risk of a badly connected leaflet anchor requiring the procedure to be aborted and started over, and this reduced risk has clear benefits for the efficiency of the procedure as well as for the health of the patient. In addition the retractable feature may allow the physicians to load and reload the catheter device with leaflet anchors more easily. A reloading operation can be necessary if multiple chordae lines are needed to be placed in a single surgical procedure. The method steps during assembly of the device will also be improved.

In some examples, both of the leaflet anchor and the ejector unit are housed inside a leaflet anchor tube of the leaflet anchor deployment mechanism prior to deployment, with the ejector unit further inside the leaflet anchor deployment mechanism than the anchor. The leaflet anchor tube may have a shape that is complementary to the shape of the leaflet anchor, i.e. with a similar cross-sectional shape. In some examples both of the leaflet anchor and the tube both have a circular cross-section with the leaflet anchor in the deformed configuration and placed into the tube. As discussed above the leaflet anchor may unfold into a hooked shape, in which case it may comprise hooks extending from a tubular body section. The ejector unit may also have a shape that is complementary to the shape of the leaflet anchor, i.e. with a similar cross-sectional shape, and this may hence also be a circular cross-section.

The leaflet anchor tube has an opening that can be directed toward the leaflet. This opening may not be at a distal end of the catheter device as a whole. In fact the opening of the leaflet anchor tube may advantageously be directed toward the proximal end of the catheter device, in order that the leaflet anchor may easily be inserted through the leaflet from the bottom of the leaflet, as is required for effective implantation of an artificial chordae line that extends from the leaflet anchor to a papillary anchor at the papillary muscle. The leaflet anchor tube may be within a gripper arrangement as disclosed in WO2016/042022 and/or may have features as described below. Thus, the leaflet anchor deployment mechanism may include a gripper for gripping the leaflet during deployment of the leaflet anchor. It can provide advantages if the catheter device combines the proposed ejector unit of this aspect with a gripper that is different to WO2016/042022 as discussed below, i.e. wherein the leaflet anchor is deployed with the gripper at an angle to the main body of the catheter device.

With arrangements using a leaflet anchor tube, the leaflet anchor may be arranged to be deployed by advancing both the leaflet anchor and the ejector unit along the tube, with the leaflet anchor having pins at its distal end that form into the hooks of a hooked shape as the pins leave the opening of the leaflet anchor tube. This can be done whilst the leaflet is gripped in a gripper of the leaflet anchor deployment mechanism as discussed above. As noted above, once the leaflet anchor is implanted then the connection can be tested in relation to position and holding strength. If needed then the leaflet anchor can be pulled back into the leaflet anchor tube to release it from the leaflet. If the connection of the anchor is acceptable then the ejector unit may be advanced further in order that the leaflet anchor is released.

Thus, in some examples, the change from the first configuration to the second configuration may be actuated by movement of the ejector unit along the leaflet anchor tube, for example by permitting the grasping device to open when it reaches a certain position in the tube. In one example the ejector unit has a constrained configuration as the first configuration, and a non-constrained configuration as the second configuration. In the first configuration the ejector unit holds the leaflet anchor with the grasping device, which may for example comprise two or more grappling hooks arranged to engage with the leaflet anchor at their ends. In one possible arrangement the grappling hooks have ends that engage with holes formed in the leaflet anchor, preferably a proximal end of the leaflet anchor with respect to the distal direction along the leaflet anchor tube. The grasping device may engage and disengage from the leaflet anchor via a radial movement relative to the leaflet anchor tube. Thus the constrained, first, configuration may involve walls of the leaflet anchor tube preventing an outward radial movement of the grasping device (such as of the grappling hooks) in order to force the ejector unit to remain engaged with the leaflet anchor. In the non-constrained, second, configuration grasping device releases the leaflet anchor, for example via the grappling hooks moving apart. The transition from the first configuration to the second configuration may occur by movement of the ejector unit to a point at which a constraint from the walls of the leaflet anchor tube is removed, so that the grasping device opens, for example by an outward radial movement of the grappling hooks. This may be due to a movement of parts of the ejector unit out of the leaflet anchor tube, i.e. out of the opening at the tube's distal end, or it may arise by movement of parts of the ejector unit to align with cut-outs in the walls of the leaflet anchor tube.

The movement of the leaflet anchor and ejector unit within the leaflet anchor deployment mechanism, for example along the leaflet anchor tube described above, can be actuated by wires and/or rods. A wire may be provided for pulling the ejector unit for retraction of the ejector unit. Retraction of the ejector unit may be required either after a successful implantation of the leaflet anchor or as part of a retraction of the leaflet anchor to allow it to be re-implanted. Since the leaflet anchor tube may be directed toward the proximal end of the catheter device, as discussed above, such that the retraction of the ejector unit requires a pulling force toward the distal end of the device, then the wire for retraction may pass around a pulley or the like. A rod may be used for deployment of the leaflet anchor, i.e. for moving the ejector unit together with the leaflet anchor along the leaflet anchor tube toward the opening at the tube's distal end. To allow for a pushing force directed toward the proximal end of the catheter device then the rod may be a U-rod. This may be arranged as described in WO2016/042022. A rod for deployment may also be capable of applying a pulling force for retraction and hence a rod may be used alone. Alternatively, the rod may be used for deployment with a wire as discussed above being used for retraction. In another alternative the ejector unit can be moved by two wires and pulleys providing for movement in both directions. The U-rod may be produced form a heat set or bent wire. With one or more bend(s) to make the U shape and the shape that pushes on the leaflet anchor.

A groove may be provided in a wall of the leaflet anchor tube for guiding the ejector unit. The groove may ensure that the ejector unit remains a single orientation relative to the tube while it is moved up and down. The groove may alternatively or additionally set maximum limits on the range of movement of the ejector unit, and thus may prevent it from going too far in either direction, out of or into the leaflet anchor tube. The ejector unit may be provided with a guide pin for engagement with the groove. Advantageously, a narrowing in the groove may be provided to act as an indicator to let the operator know when the ejector unit has reached a certain position. The size of the guide pin and the width of the narrowing may be set so that engagement of the pin with the narrowing in the groove will require an increased force before further movement can be made, thus providing tactile feedback to the operating physician.

In one example a force feedback mechanism, such as the narrowing, is provided in order to signify that the leaflet anchor has been moved to the deployed position, but that the ejector unit is still in the first configuration so that the anchor is still retractable. In this case, once the ejector unit is pushed further (e.g., so that the guide pin is beyond the narrow section) then the ejector unit may move to the second configuration so that the leaflet anchor will be released from the ejector unit. Thus, in one example constrained parts of the ejector unit, such as the grappling hooks discussed above, may be released from their constraint once there is movement beyond a point of actuation of the force feedback mechanism, such as when the guide pin passes the narrowing in the example above. Alternatively or additionally there may be feedback mechanisms in the operation handles of the catheter device that can indicate the position of the ejector unit, for example by varying forces or by visual indicators. In an alternative to a guide pin and narrowing groove system another form of force feedback mechanism may act on the guide pin, for example a "shear-pin" suture that breaks at a given point with a given load.

The leaflet anchor deployment mechanism may include a line pusher for directing a line out of and away from the leaflet anchor deployment mechanism during deployment of the leaflet anchor. When the device is in use there may be a line attached to the leaflet anchor. The line may be provided to form the artificial chordae line after the leaflet anchor is implanted, or to allow the artificial chordae line to be attached to the leaflet anchor. The line may be a suture such as a Goretex ePTFE suture. The line pusher advantageously directs the line away from the leaflet anchor deployment mechanism so that it can be more readily accessed for later manipulation, such as for tightening the line or for pulling on the implanted leaflet anchor for testing of the connection. The line pusher may be actuated during the action of deployment of the leaflet anchor, and in some examples it is actuated when the leaflet anchor is released from the ejector unit. Thus, the line pusher may be released when the ejector unit withdraws away from the implanted leaflet anchor. The line pusher may transition from a constrained state to a non-constrained state in a similar way to the grappling hooks described above, and thus it may move radially outward to push the line out, with this radially outward movement being permitted and the line pusher released once a constraint is removed. The constraint may be from the leaflet anchor, and thus the constraint may be removed, when the ejector unit is pulled back into the leaflet anchor deployment mechanism. In that case the line pusher may be an arm that extends axially forward from the ejector unit toward the leaflet anchor, and radially outward of the leaflet anchor tube when the arm is at rest with no forces applied. Prior to deployment of the leaflet anchor the arm of the line pusher is bent elastically to place its distal end within the leaflet anchor, so that it is constrained and cannot move to its radially outward position until the leaflet anchor and the ejector unit move apart. In some examples, as the ejector unit continues to withdraw into the leaflet anchor deployment mechanism the line pusher may remain in its unconstrained state with the line pusher as well as the line being pushed out of a slit in the leaflet anchor deployment mechanism, such as a slit along the leaflet anchor tube.

The catheter device of the fifth aspect may further be provided with a papillary anchor and papillary anchor deployment mechanism for deployment of a papillary anchor for attachment to the papillary muscle. The papillary anchor deployment mechanism may be arranged for deployment of the papillary anchor by moving it outward in the distal direction relative to the distal part. The papillary anchor deployment mechanism may be arranged within a two-part housing section as discussed above with reference to the fourth aspect, in which case the leaflet deployment mechanism may be in the proximal part of the two-part housing section. Alternatively, the papillary anchor deployment mechanism may be similar to that described in WO2016/042022. In some examples the actuation of the leaflet anchor may be connected to the papillary anchor deployment, meaning that the leaflet and papillary anchor may be arranged to be at least party deployed at the same time, for example being actuated by a single control wire or rod. This can make the procedure easier and/or faster.

The papillary anchor deployment mechanism may include a lock that prevents the papillary anchor from ejecting too early, which may happen if the outer shaft that holds the device is compressed, while the inner papillary anchor deployment shaft is stretch or keeps it original length while the outer shaft is shortened, pushing the papillary anchor out of its housing. The lock may be a flip out tab that holds the anchor adjustment and ejector mechanism in place, the tab may be operated with a torque, push or pull wire or a suture. The actuation wire/suture may be routed through the gripper housing and supported there or supported in the papillary housing, alternatively anchored in the anchor deployment mechanism itself. In a second configuration the locking mechanism may sit inside the papillary anchor deployment mechanism and be actuated by a wire that goes inside the adjustment catheter. As noted above, wire(s) and/or a rod can be used to deploy and/or retract the ejector unit. In another variation the ejector unit may be moved via a sliding sheath that engages with a lug on the ejector unit. This sheath may fit around the leaflet anchor tube. The sheath may be a partial tube, such as a three quarter tube, that goes around the leaflet anchor tubing. Such an arrangement may also be called "sledge", or a "linear motion bearing". The sheath when moved will push on the lug of the ejector unit. The sheath may be actuated by one or more wire(s) or rod(s), which may be connected with a rotational joint to the sheath. For example, there may be one or more wires that can be pulled or pushed by the operator. Nitinol wires may be used. When pulled or pushed the sheath translates along the outside of the leaflet anchor tube, for example to move towards the opening of the tube and push the ejector unit via the lug. The lug may be the guide pin in the groove as discussed above.

The ejector unit and/or the leaflet anchor may be produced from an elastic metal, such as nitinol. The ejector unit and/or the leaflet anchor may be laser cut, heat set and electropolished metal tubing. The guide pin and/or lug, where present may be welded into place after assembly, such as by laser welding. The grappling hooks of the ejector unit may be heat set or laser welded in place, and they may have any suitable shape for engagement with the leaflet anchor. The leaflet anchor tube may be attached to the leaflet anchor deployment mechanism, such as attachment to the gripper, by welding, soldering or gluing, or it could be cut from a solid piece via subtractive manufacturing. Additive manufacturing techniques might also be used. Additional tubes may also be provided next to the leaflet anchor tube, for example to provide fluid flow or for covering wires. At the end of the leaflet anchor tube there may be a gripper tip that extends laterally around the leaflet anchor tube to form a gripping platform that fits with an opposing gripper element of the leaflet anchor deployment mechanism. The gripping platform may be formed by filling an end of the gripper with resin. The leaflet anchor tube may have a lever arm attached, such as a heat set(or squashed) flat section or a bent section, wherein the lever arm stretches past a rotation axis (the rotation axis may move during the gripper arms movement) of the gripper to attach wires used to open and/or close the gripper.

The leaflet anchor tube may be laser-welded to a gripper tube section, inside the chordae slit. Further features of possible gripper arrangements may be similar to those disclosed in WO2016/042022 and/or may be as set out below.

Viewed from a sixth aspect, there is disclosed a method of use of the catheter device of the fourth aspect for implanting both of a leaflet anchor and a papillary anchor into the heart during a procedure for implanting an artificial chordae line that extends between the leaflet anchor and the papillary anchor, wherein the method comprises: deployment of the leaflet anchor into the leaflet using the leaflet anchor deployment mechanism; angling the flexible joint in order to bring the papillary anchor deployment mechanism into close proximity with the papillary muscle (optionally, alternatively or additionally, extending the joint if it is extendable); and deployment of the papillary anchor into the papillary muscle using the papillary anchor deployment mechanism. This method may include use of a device with any of the other features discussed above with reference to any of the various device aspects, and/or method features as discussed below. The method may include testing the connection of the leaflet anchor prior to deployment of the papillary anchor, such as via testing as discussed above.

Viewed from a seventh aspect, there is disclosed a method of use of the catheter device of the fifth aspect for implanting a leaflet anchor into the heart during a procedure for implanting an artificial chordae line, the method comprising: deployment of the leaflet anchor into the leaflet using the leaflet anchor deployment mechanism with the ejector unit initially remaining in its first configuration; and later movement of the ejector unit into the second configuration to thereby release the leaflet anchor. The method may advantageously include testing the connection of the leaflet anchor before moving the ejector unit from the first configuration to the second configuration, such as via testing as discussed above. The method may include, if the connection of the leaflet anchor is found to be inadequate, keeping the ejector unit in the first configuration, withdrawing the leaflet anchor into the leaflet anchor deployment mechanism using the ejector unit and later re-deploying the leaflet anchor using the leaflet anchor deployment mechanism before testing the connection again. This can be repeated until an adequate connection is achieved, at which point the ejector unit should be moved from to the second configuration to release the leaflet anchor. This method may include use of a device with any of the other features discussed above with reference to any of the various device aspects, and/or method features as discussed in relation to the fourth or sixth aspect above, or the other aspects below.

Viewed from an eighth aspect, there is disclosed a method of use of the catheter device of the third aspect for repair of the heart by implanting an artificial chordae line, the method comprising: moving the second gripper arm away from the main body of the catheter device; moving the first gripper arm away from the main body of the catheter device; at least one of: rotating the first gripper arm to bring it into contact with the second gripper arm to thereby grasp the leaflet at a point spaced apart from the main body of the catheter device; rotating the first gripper arm to bring it into contact with the second gripper arm to thereby restrain the leaflet before rotating the gripper arm to grasp the leaflet between the first gripper arm and the main body of the catheter device; and pushing the leaflet anchor out of the leaflet anchor tube to pierce the leaflet and form the leaflet anchor into an unfolded configuration so that hooked formations of the leaflet anchor secure the leaflet anchor in the leaflet. This method may include use of a device with any of the other features discussed above with reference to any of the various device aspects, and/or method features as discussed in the method aspects herein.

Viewed from a ninth aspect there is disclosed a method of use of the anchor of the second aspect for affixing an artificial chordae line to the heart, the method comprising using an anchor deployment device to implant the anchor into the tissue of the heart. The anchor may be used as a leaflet anchor with the method hence including the use of a leaflet anchor deployment mechanism. This method may include use of a device with any of the other features discussed above with reference to any of the various device aspects, and/or method features as discussed in the method aspects herein. The method may include testing the connection of the anchor to the tissue of the heart, such as via testing as discussed above.

Viewed from a tenth aspect there is disclosed a method of manufacture of the catheter device of the fourth aspect, the method comprising forming the flexible and optionally extendable joint via cutting of an elastic metal tube. Optionally the same elastic metal tube is also used to form the distal and proximal parts of the two-part body section, which are hence integrally formed with the flexible joint. A nitinol tube may be used and/or the cutting step may use laser cutting. The laser cut tube may be electropolished after cutting in order to remove any sharp edges.

It is considered to offer particular benefits to be able to form the device of the fourth aspect using the method of the tenth aspect, although it should be noted that other manufacturing methods may be used as discussed above. The method of the tenth aspect may include providing the catheter device with any of the features discussed above with reference to the various device aspects.

Viewed from an eleventh aspect there is disclosed a method of manufacture of the ejector unit for the catheter device of the fifth aspect, the method comprising: forming tines into an elastic metal tube via cutting; and deforming the end of the tines with heat setting in order to form a hooked configuration. The ejector unit may be provided with features as discussed above in connection with optional features of the fifth aspect. The manufacturing method may include providing a catheter device as in the fifth aspect and inserting the ejector unit into the catheter device along with a leaflet anchor. A nitinol tube may be used and/or the cutting step may use laser cutting. The laser cut tube may be electropolished after cutting in order to remove any sharp edges.

Viewed from a twelfth aspect there is disclosed a method of manufacture of the catheter device of the third aspect, the method comprising forming a hinge of the first gripper arm integrally with the main body of the catheter device via cutting of an elastic metal tube. The method may optionally include forming the entirety of the first gripper arm, including the hinge, integrally with the main body. It is considered to offer particular benefits to be able to form the device of the third aspect in this way, although it should be noted that other manufacturing methods may be used as discussed above. A nitinol tube may be used and/or the cutting step may use laser cutting. The laser cut tube may be electropolished after cutting in order to remove any sharp edges. The method of the tenth aspect may include providing the catheter device with any of the features discussed above with reference to the various device aspects. This method may be combined with the method of the tenth aspect in order to form a single unitary body section with the hinge of the first gripper arm (and optionally also the remainder of the first gripper arm) formed in the same integral section as the two-part housing section with the distal part and proximal part connected by the flexible joint.

Viewed from a thirteenth aspect the invention provides a method of manufacture of the anchor of the second aspect, the method comprising: forming the anchor from an elastic material; and joining the line to the anchor. The method may comprise: forming tines into the elastic metal tube via cutting; forming openings in the tines; and deforming the tines into hooked forms and heat setting them to form the hooks with openings. The anchor may be provided with features as discussed above in connection with optional features of the second aspect. It is considered to offer particular benefits to be able to form the anchor of the second aspect in this way, although it should be noted that other manufacturing methods may be used as discussed above. A nitinol tube may be used and/or the cutting step may use laser cutting. The laser cut tube may be electropolished after cutting in order to remove any sharp edges.

In any of the aspects discussed above, the leaflet anchor may be formed from an elastic material and to be arranged so that it assumes an unfolded configuration when no force is applied, and to be able to deform elastically into a folded configuration, for example when constrained within a leaflet anchor tube. The leaflet anchor may be made of a shape memory material, for example a shape memory metal. Nitinol may be used for the leaflet anchor. In some example embodiments the leaflet anchor is made from a laser cut nitinol tube. The anchor may be subject to electropolishing after laser cutting in order to remove undesirably rough or sharp edges. The edges may be chamfered before electropolishing in order to introduce greater curvature, e.g. where sutures or wires may bear against the edges when the anchor is in use.

One exemplary form for the leaflet anchor of any of the above aspects is a grapple hook shape, when it is in the unfolded configuration. The leaflet anchor may hence comprise a straight central shaft with a number of hooks spaced apart radially around the shaft. When in the folded configuration the hooks would be straightened out. The leaflet anchor may conveniently be manufactured by cutting a tube to form sharpened tines at one end, which are then bent into the hooks, with the other end of the tube forming the shaft. The shaft may have a diameter that is relatively small compared to the radial extent of the hooks in the unfolded configuration. For example the shaft may have a diameter of 30% or less of the maximum radial extent of the hooks, for example 20% or less. In one example the shaft is 1-2mm in diameter and the hooks extend over a diameter of about 5-25mm. If a shape memory material such as nitinol is used then the tines may be bent and heat set into the grappling hook shape after laser cutting of the nitinol tube.

The leaflet anchor may be provided with one or more sheaths of biocompatible material around the hooks, for example a sheath of ePFTE. This material may be placed around the majority of the hooks leaving the ends of the hooks free so as not to impede piercing of body tissue. A single sheath may be used to provide a covering for two hooks by means of cut outs allowing the sheath to extend across the centre of the anchor and be threaded onto two hooks at two sides of the anchor. Such a sheath might be a tube with an opening, or multiple openings, along one side of the tube where it bridges the centre of the anchor, thus allowing the two hooks to be threaded into the opening(s) at two sides of the centre. A method of manufacture of such a hook with a sheath may comprise inserting the hooks of the anchor into one or more sheaths, e.g. by threading the hook into an ePTFE tube or tubes. An added benefit with this approach is that the artificial chordae line may be threaded around the sheath, locking it in place in the centre of the anchor. This is not possible if the hooks are threaded with individual tubes and/or sheaths, and it allows for easier routine of the line.

Viewed from an fourteenth aspect, there is disclosed a method of use of the anchor of the second aspect for affixing an artificial chordae line to the heart, the method comprising using an anchor deployment device to implant the anchor into the tissue of the heart. The anchor may be used as a papillary anchor with the method hence including the use of a papillary anchor deployment mechanism. Alternatively, the anchor may be used as a leaflet anchor with the method hence including the use of a leaflet anchor deployment mechanism. This method may include use of a device with any of the other features discussed above with reference to any of the various device aspects, and/or method features as discussed above in the other method aspects. The method may include testing the connection of the anchor to the tissue of the heart, such as via testing as discussed above.

In relation to any of the aspects discussed above, it is advantageous if the leaflet anchor can be placed into the leaflet from beneath, i.e. from the side where the papillary muscle is located, so that the new artificial chordae line may pull the leaflet downward. However, the most convenient route to access the heart involves the catheter entering from above the leaflet. To facilitate the placement of the leaflet anchor from beneath, the catheter device of any of the above aspects may be arranged so that the open end of a leaflet anchor tube is at a proximal end of the gripper device (the 'upper' end when in the heart in the above defined orientation) and the leaflet anchor can be pushed out of the tube moving from the distal end of the catheter device toward the proximal end. The catheter device may include a U-shaped rod for deployment of the leaflet anchor. This may be a U-shaped piece at the end of a wire that is used to actuate the leaflet anchor. Alternatively it may be a U-shaped rod attached to a separate wire at one end of the U-shape. In either arrangement the free end of the U-shape abuts the end of the leaflet anchor and is arranged to push the anchor toward the proximal end of the catheter device when the wire is pulled. The U-shaped rod should be sufficiently stiff to hold its shape when pulled with force applied to the anchor. A ball may be placed at the free end of the U-shaped rod to allow it to best engage with the leaflet anchor (or with the ejector unit, where present). In this way the leaflet can be pierced from beneath.

When the leaflet anchor tube is in the gripper arm, such as the first gripper arm, then the U-shaped rod may extend into the gripper arm. In this case the U-shaped rod needs to be sufficiently elastic to bend when the gripper arm is opened and closed. The U-shaped rod may have a flexible section, for example a section of narrowed cross-section, for aiding the bending motion. The U-shaped rod may also or alternatively be made of a suitably elastic material, which could be nitinol. Advantageously, the elasticity of the U-shaped rod may act as a spring to return the gripper arm to the closed position.

Alternatively the U-rod wire may be made so that no bending is necessary while the gripper opens, if the end of the U-rod is small enough to not touch the walls of the leaflet anchor tube while the gripper rotates, it does not have to bend while the gripper opens. This advantageously allows for a greater operation angle not limited by a requirement for allowing for the U-rod to deflect.

The line may be an artificial chordae line. The artificial chordae line may be a Gore-Tex^{®} suture or other appropriate biocompatible material, such as a thin nitinol wire, an ultrahigh-molecular-weight polyethylene (UHMWPE) wire, or a composite wire comprising a tough core such as nitinol or high strength suture and an outer coating such as PTFE or ePTFE. The artificial chordae line may comprise an ePTFE suture tube, which may be threaded with a Dyneema core. This Dyneema core may be the same suture that is threaded through the leaflet anchor as mentioned above. The ePTFE-Dyneema tube construction of the artificial chorda line may in addition be coupled to a wire (preferably nitinol) in the opposite end of the leaflet anchor, for example by threading the nitinol wire into the ePTFE tube together with the Dyneema core. The ePTFE tube and the Dyneema wire can then be joined to the anchor by the knotting configuration as discussed above to allow adjustment of the new artificial chordae line with minimal friction. Such adjustment may be done through an adjustment catheter. In some example embodiments the catheter device also holds a papillary anchor for attachment to the papillary muscle. The artificial chordae line may extend from the leaflet anchor to the papillary anchor. In some embodiments the artificial chordae line joins the two anchors together directly, with no intervening clip as in WO 2008/101113. This means that the artificial chordae line can more closely emulate the natural chords, and so the repair to the heart is more effective.

Certain example embodiments of the invention will now be described by way of example only and with reference to the accompanying drawings in which:
Figure 1 illustrates the procedure for insertion of a catheter device through a mitral valve;
Figures 2 to 6 show the action of a mechanical gripping mechanism using two gripper arms;
Figure 7 illustrates gripping of a leaflet of the mitral valve with one gripper arm;
Figures 8 to 12 show deployment of a leaflet anchor in a device using an ejector device;
Figure 13 shows a close up view of the valve during placement of a leaflet anchor, which is coupled to an artificial chordae line;
Figure 14 shows movement of the distal end of the catheter device to the papillary muscle for placement of a papillary anchor;
Figure 15 illustrates withdrawal of a treatment catheter part of the device and adjustment of the chord length with an optional adjustment catheter;
Figures 16 and 17 show an example of a hook for an anchor which is threaded with a suture;
Figures 18 and 19 show the folded and unfolded configuration of an example of a papillary anchor;
Figure 20 is a cross-section through a lower (distal) part of the main body of the catheter device showing how the main parts fit inside a papillary anchor deployment mechanism;
Figure 21 shows an example arrangement for the routing of the artificial chordae line and other lines within the papillary anchor deployment mechanism of Figure 20;
Figure 22 is a cross-section of an example with the papillary anchor deployment mechanism of Figure 20 and a gripping mechanism as in Figures 2 to 6, including one possible routing of the artificial chordae line between the papillary anchor and the first gripper arm;
Figure 23 shows an anchor in combination with a line;
Figure 24 shows an anchor in combination with a line implanted in body tissue; and
Figure 25 shows an anchor in combination with a line.

The catheter devices presented here are proposed for non-surgical (endovascular) insertion of mitral chords to address mitral regurgitation caused by prolapse of a leaflet 12 of the valve. The Figures show different forms of catheter device 2 for this purpose, but it will be understood that the general principles are the same for each device in terms of implantation of a leaflet anchor 10 and a papillary anchor 9 in order to insert one or more artificial chordae lines 14 into the heart. The artificial chordae line(s) 14 are fixed to the prolapsing leaflet 12 and to the papillary muscle 26, thereby recreating a normal anatomy. A single catheter device 2 is used to place both a leaflet anchor 10 and a papillary anchor 9. The length of the chord 14 can be adjusted, again using the same catheter device 2, to eliminate the mitral regurgitation. Thus, the new device enables a single minimally invasive endovascular procedure to be used to repair the mitral valve, providing significant advantages compared to earlier systems requiring more invasive procedures and/or multiple operations.

It should be noted that although an endovascular approach is preferred and the device is hence capable of using this approach, the device could of course be used in different procedures, including more invasive procedures. Many of the advantages will remain, and it could be beneficial to use this device in situations where a more invasive procedure is merited. In addition, it is contemplated that, as discussed above, aspects of the design of the papillary anchor 9 could be used for an anchor for other purposes and this disclosure is not intended to be limited in this regard.

The catheter device 2 described in the following can be used to insert mitral chords through the venous system, starting in the femoral vein in the groin. A catheter is advanced to the right atrium. Approach to the left atrium is then gained by a so-called transseptal puncture whereafter a larger guidance catheter is advanced into the left atrium. The catheter device 2 for the heart repair is then introduced through the guiding catheter and into the left atrium.

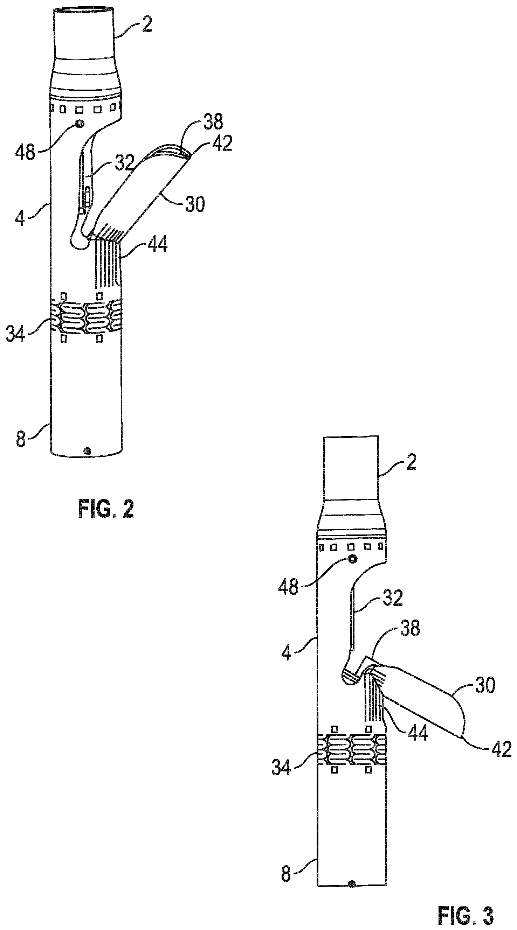

X-ray and ultrasound guidance is used to position the device and, as explained in more detail below, the mitral leaflet 12 is grabbed and a new artificial chordae line 14 is attached using a self-expandable leaflet anchor 10. The artificial chordae line 14 is then attached to the papillary muscle 26, using a, papillary anchor 9. Advantageously, the catheter device shown in Figures 2 to 6, 14 and 20 to 22 can be used to place the papillary anchor 9 whilst the leaflet 12 is still being grasped by the device. The chord length can now be adjusted to eliminate any mitral regurgitation. Excess chord is then cut and all catheters are withdrawn. Echo and Doppler imaging is used to perform the procedure and monitor the result. The successful use of this endovascular technique will drastically reduce the invasiveness, complications and cost of mitral valve repair.

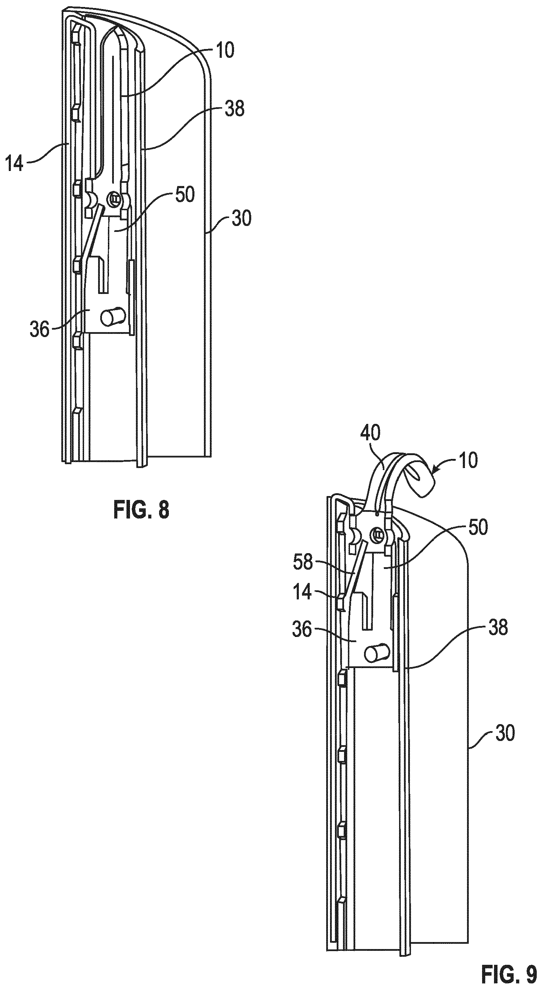

More detail on the structure and function of the device is set out below with reference to the Figures. The procedure of using one form of the device can be summarised as follows:
1) The femoral vein is entered using standard Seldinger technique and the guiding catheter introduced.
2) The guiding catheter is advanced to the right atrium under x-ray guidance.
3) The left atrium is entered after penetration of the atrial septum, guided by x-ray and transesophageal echo.
4) Correct position of the entrance site in the left atrium is verified to assure proper alignment for insertion of the guiding and treatment catheters. The entrance hole in the atrial septum is dilated and the guiding catheter is advanced into the left atrium.
5) A treatment catheter device 2 is advanced through the guiding catheter and positioned in the left atrium above the mitral valve.
6) The prolapsing segment of the mitral leaflet 12 is located with ultrasound and the treatment catheter device 2 is advanced into the left ventricle placing a gripper 6 of the treatment catheter device 2 in position to grip the prolapsing segment. Advantageously, this may use a gripper 6 with two gripping arms 30, 32 as discussed in more detail below with reference to Figures 2 to 6.
7) The prolapsing segment is gripped and after assuring correct position the leaflet anchor 10 is pushed through the leaflet 12 allowing it to open and fix the leaflet 12.
8) The connection of the leaflet anchor 10 may be tested whilst it remains attached to the catheter device 2 via an ejector unit 36 as discussed further below with reference to Figures 8 to 12, and if the connection is sufficient then the distal end of catheter is advanced further into the left ventricle, advantageously using a flexible and extendable joint 34 as shown in Figures 2 to 6 and 14, or using a flexible joint as shown in Figures 31 and 32 to angle the joint without extension, until the distal end makes contact with the papillary muscle 26 or surrounding tissue.
9) The papillary anchor 9 is pushed into the papillary muscle 26 area and out of its housing 8 thereby letting the papillary anchor 9 open inside the papillary muscle 26.
10) If the gripper 6 is still grasping the leaflet 12 then it is released, such as by releasing the leaflet anchor 12 from the ejector unit 36 as discussed below with reference to Figures 8 to 12.
11) The length of the artificial chordae line 14 is adjusted until mitral regurgitation is eliminated.
12) The catheter device 2 is pulled back from the papillary anchor 9, and elimination of mitral regurgitation is again confirmed by echocardiography.
13) The position of the artificial chordae line 14 is locked at the papillary anchor 9.
14) The excess chordae line 14 is cut.
15) Additional artificial chordae lines may be placed if necessary.
16) The catheter device is fully withdrawn and removed from the vascular system.

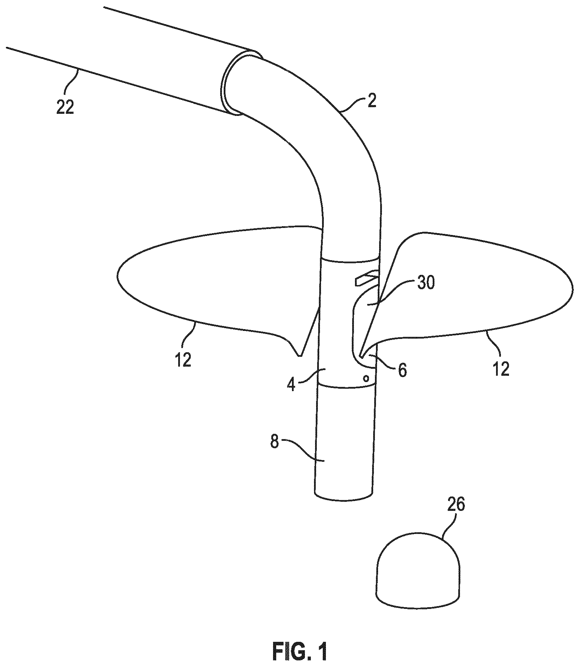

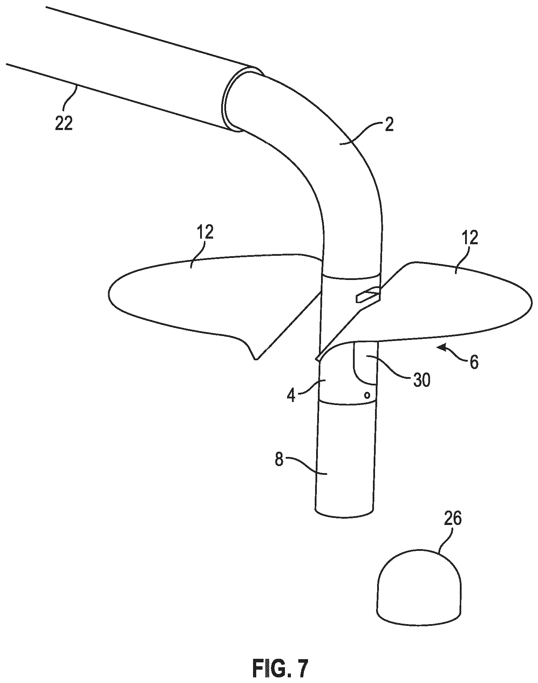

Figure 1 shows guide catheter 22 that has been used to steer a catheter device 2 to a required position within the heart adjacent extending through the mitral valve and hence being between two leaflets 12. The catheter device 2 is composed of four different main parts; a steerable catheter, a gripper housing 4, a gripper device 6 and a papillary anchor housing 8, which holds a papillary anchor 9. Advantageously the gripper housing 4 and the papillary anchor housing 8 may form a proximal part 4 and a distal part 8 of a two part housing section with a central flexible and extendable joint 34 as shown in Figures 2 to 6, 14 and 20 to 22. Thus, it should be understood that the procedure shown in Figure 1 (and likewise in Figures 7, 13 and 15) may use this arrangement for the gripper housing (proximal part) 4 and papillary anchor housing (distal part) 8. The steerable catheter could be replaced with an alternative arrangement using a steerable sheath about a steerable catheter and flexible tubing within the steerable catheter.

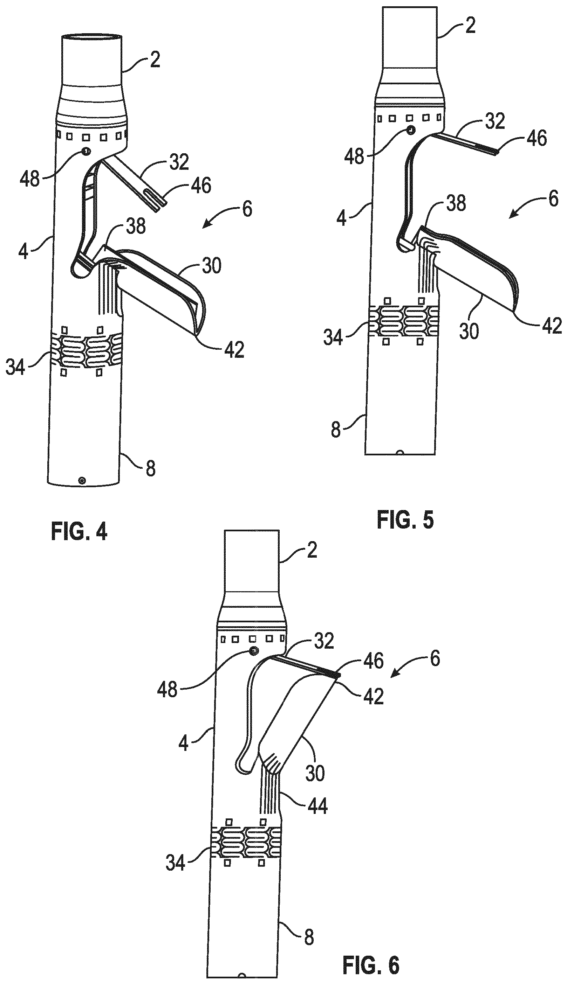

Figure 1 shows a front view of one example catheter device with the gripper device 6 closed. The gripper device 6 of some arrangements uses a single gripper arm 30 that grips the leaflet 12 against the gripper housing part 4 as shown in Figure 7. In other arrangements the gripper device 6 uses two gripper arms 30, 32 as shown in Figures 2 to 6 in order to allow the leaflet 12 to be grasped between the two gripper arms 30, 32 at a point spaced apart from the main body of the catheter device. The gripper device 6 is a part of a leaflet anchor deployment mechanism for deploying the leaflet anchor 10 to attach it to the leaflet 12 of the heart. The gripper device 6 includes a leaflet anchor tube 38 for housing the leaflet anchor 10 in a folded configuration prior to deployment. In the example embodiments the leaflet anchor tube 38 is in the (first) gripper arm 30, as seen in Figures 2 and 4, for example. When the gripper device 6 grasps the leaflet 12, the leaflet anchor 10 can be pushed out of the leaflet anchor tube 38 to pierce the leaflet 12 and form the leaflet anchor 10 into an unfolded configuration so that hooked formations 40 of the leaflet anchor 10 secure it in the leaflet 12.

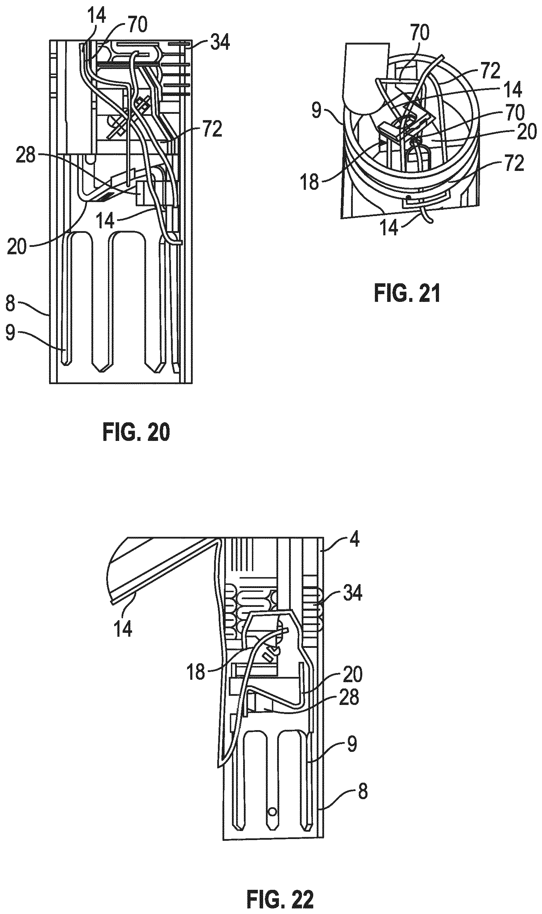

The leaflet anchor 10 is connected to an artificial chordae line 14, which can sit inside a narrow channel that goes along the surface of the first gripper arm 30 (as shown in Figures 8 to 12, for example) and via the papillary anchor housing 8 to the papillary anchor 9 (as shown in Figures 20 to 22, for example). The channel can be slightly smaller than the diameter of the new artificial chordae line 14 and/or have a thin shielding structure (not shown). This makes the artificial chordae line 14 sit in place due to a friction fit. The new artificial chordae line 14 goes into the papillary anchor housing 8 and through a papillary anchor locking section, through a locking and cutting piece 18, and through Z shaped fork 20. These parts are described in further detail below with reference to Figures 20 to 22. The new artificial chordae line 14 can be attached to a wire which passes back along the catheter all the way to the outside (to make the adjustment smoother). The wire allows for a shortening of the chord during the procedure, by pulling, or a lengthening of the chord, since the wire can be pushed through the catheter.

The two-part housing section, with the gripper housing (proximal part) 4 and papillary anchor housing (distal part) 8 might be approximately 6-7 mm in diameter, and approximately 30 mm in length.

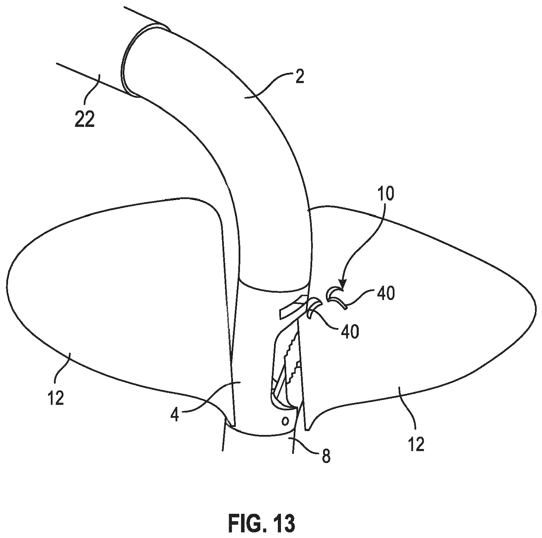

Figures 2 to 6 show steps in movement of the gripper mechanism 6 in an example with two gripper arms 30, 32 as discussed above. This gripper mechanism 6 is a part of a housing section that also includes a flexible and extendable joint allowing the papillary anchor housing 8 (distal part) to be moved toward the papillary muscle 26 after the leaflet 12 has been grabbed by the gripper mechanism 6. In this example, in order to grasp the leaflet 12, the first gripper arm 30 is rotated to move its end 42 away from the main body of the catheter device, with this rotation being enabled via a weakened area 44 of the tubular form of the main body. It can be seen that the leaflet anchor tube 38 sits inside the first gripper arm 30, with the end of the leaflet anchor tube 38 having an opening at the end 42 of the first gripper arm 30. With the first gripper arm 30 open, the second gripper arm 32 is free to rotate to move its end 46 outward of the main body. In this example the second gripper arm 32 rotates around a hinge formed by pins 48 placed in holes in the proximal part 4 of the two-part housing section, but it will be appreciated that a similar final placement of its end 46 may be achieved via a sliding movement. With the second gripper arm 32 folded outward the first gripper arm 30 can close so that the two ends 42, 46 come into contact at a point spaced apart from the main body of the device. This allows the leaflet 12 to be grasped. With the leaflet 12 in place the leaflet anchor 10 can be moved out of the leaflet anchor tube 38 to implant it, such as via a mechanism with an ejector unit 36 as described below in relation to Figures 8 to 12, with the final positioning of the leaflet anchor 10 being similar to that shown in Figure 13.

Figure 7 shows an alternative form of gripper mechanism 6 that grasps the leaflet 12 with a single gripper arm that holds it against the gripper housing 4. This could also use the ejector unit 36 mechanism of Figures 8 to 12.

A ridged surface on the gripper arm(s) 30, 32 may be provided to help it grip the leaflet 12. 3D ultrasound and/or other available sources can be used to confirm that the gripper mechanism 6 has grasped the correct part of the leaflet 12.

The gripper mechanism 6 can be opened and closed as many times as needed to grasp the right part of the leaflet 12. The opening and closing may be facilitated by a system allowing for one wire to pull the gripper mechanism 6 open, and one to pull it closed. Different arrangements of wires and/or rods may be used to control the example with two gripper arms 30, 32, as discussed above. Once the position of the gripper mechanism 6 is confirmed then the leaflet anchor 10 can be pushed out of the end of the leaflet anchor tube 38, such as by pulling a wire in the other end of the catheter. Figure 13 shows a close up view of the leaflet anchor 10 placed in the leaflet 12 with the hooked formations 40 engaging with the leaflet 12.

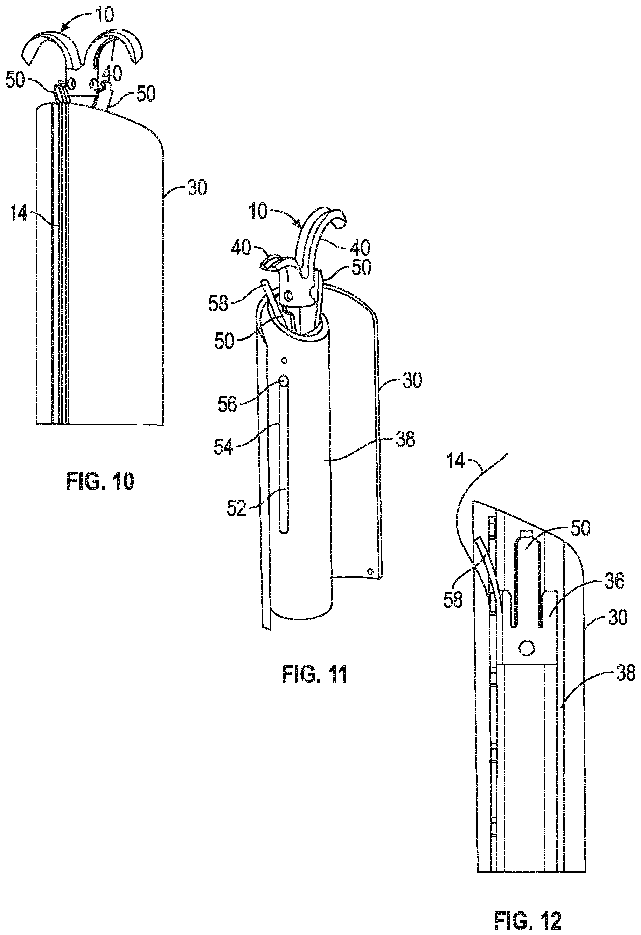

As noted above, an ejector unit 36 may be used as shown in Figures 8 to 12. With the use of the ejector unit 36 the leaflet anchor deployment mechanism allows for retraction and repositioning of the leaflet anchor 10 after deployment of the anchor 10 into the leaflet 12. This is achieved via the ejector unit 36, which includes a grasping device 50 with a first configuration, as shown in Figure 8 and Figure 9 and a second configuration as shown in Figure 10 and Figure 11.

In the first configuration the grasping device arranged to permit deployment of the leaflet anchor 10 into the leaflet 12 without disengagement of the leaflet anchor 10 from the ejector unit 36. Thus, the grasping device 50, which in this example comprises two grappling hooks 50 as shown, grips the leaflet anchor 10 and can advance along the leaflet anchor tube 38 from the fully stowed position as in Figure 8, to a position in which the anchor 10 is deployed as shown in Figure 9, without releasing the anchor 10. The grappling hooks 50 are held to the leaflet anchor 10 as they are constrained within the leaflet anchor tube 38. The ejector unit 36 is hence arranged so that it remains in the first configuration whilst the leaflet anchor 10 is being implanted. With the leaflet anchor 10 implanted the grasping device 50 and ejector unit 36 can be used to test the connection of the leaflet anchor 10 to the leaflet 12, for example by a force being applied to the leaflet anchor from the ejector unit whilst the grasping device 50 is in the first configuration.

The grasping device 50 moves into the second configuration when the constraint from the leaflet anchor tube 38 is no longer present, for example when the grappling hooks 50 move beyond the end of the tube as shown in Figure 10. Thus, if the connection has been tested and the physician decides to release the leaflet anchor 10 then they can further advance the ejector unit 36, which will move it into the second configuration. In this second configuration the grasping device 50 of the ejector unit 36 is disengaged from the leaflet anchor 10.

If the physician is not satisfied by the connection during the testing (for example, if there is too much movement of the anchor 10 and/or not enough resistance to force on the line) then the leaflet anchor 10 can be retracted and placed in another location. If the grasping device 50 did not change from the first configuration during this test then the latter procedure may be carried out by reversing the deployment of the ejector unit 36 and leaflet anchor 10, for example by drawing those parts back into the leaflet anchor deployment mechanism. If the second configuration was used before it was determined that the connection of the anchor was not adequate then to retract the anchor 10 the ejector unit 36 should be first moved back to the first configuration so that the grasping device 50 reengages with the leaflet anchor 10, and then after that the deployment of the ejector unit 36 and leaflet anchor 12 is reversed, for example by drawing those parts back into the leaflet anchor tube 38.

A groove 52 is provided in a wall of the leaflet anchor tube 38 for guiding the ejector unit 36. The groove 52 ensures that the ejector unit 36 remains a single orientation relative to the tube 38 while it is moved along the tube. The groove 52 can set maximum limits on the range of movement of the ejector unit 36 and thus may prevent it from going too far in either direction, out of or into the leaflet anchor tube 38. The ejector unit 36 has a guide pin 56 for engagement with the groove 52. A narrowing 54 in the groove 52 is provided to act as an indicator to let the operator know when the ejector unit 36 has reached a certain position. The size of the guide pin 56 and the width of the narrowing 54 are set so that engagement of the pin 56 with the narrowing 54 in the groove 52 will require an increased force before further movement can be made, thus providing tactile feedback to the operating physician.

The leaflet anchor deployment mechanism of Figures 8 to 12 also includes a line pusher 58 for directing the artificial chordae line 14 out of and away from the leaflet anchor tube 38 during deployment of the anchor 10. The line pusher 58 directs the artificial chordae line away from the leaflet anchor tube 38 so that it can be more readily accessed for later manipulation, such as for tightening the line 14 or for pulling on the implanted leaflet anchor 10 for testing of the connection. The line pusher 58 is actuated during the action of deployment of the leaflet anchor 10, with this actuation being triggered when the leaflet anchor 10 is released from the ejector unit 36. Thus, the line pusher 50 is released when the ejector unit 36 withdraws away from the implanted leaflet anchor 10.

In the example shown, the line pusher 58 transitions from a constrained state to a non-constrained state and moves radially outward to push the line 14 out, with this radially outward movement being permitted and the line pusher released once a constraint from the leaflet anchor 10 is removed. The line pusher 58 is an arm that extends axially forward from the ejector unit toward the leaflet anchor 10 and radially outward of the leaflet anchor tube 38 when the arm is at rest with no forces applied. Prior to deployment of the leaflet anchor 10 the arm of the line pusher 58 is bent elastically to place its distal end within the leaflet anchor 10, as shown in Figures 8 and 9, so that it is constrained and cannot move to its radially outward position until the leaflet anchor 10 and the ejector unit 36 move apart, as is best shown in Figure 11. As the ejector unit 36 continues to withdraw into the leaflet anchor tube 38 the line pusher 58 remains in its unconstrained state with the line pusher 58 as well as the line 14 being pushed out of a slit in the leaflet anchor tube 38, as shown in Figure 12.

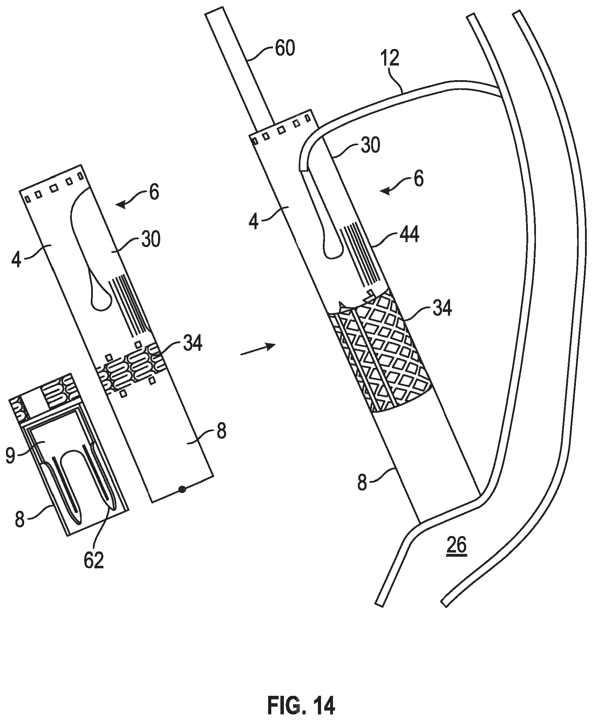

With the leaflet anchor 10 implanted in the leaflet 12 the papillary anchor housing 8 at the end of the treatment catheter is then placed onto the papillary muscle 26. With the use of a flexible and extendable joint 34 this may be done as shown in Figure 14. In this example, the flexible and extendable joint 34 is formed by flexible meandering sections cut into a tubular form of the main body. Advantageously the flexible and extendable joint 36 is formed integrally with a tubular distal part 8, which provides the papillary anchor housing 8 and with a tubular proximal part 4, which provides the gripper housing 4. Further advantageously the tubular form of the gripper housing 4 may include an integrally formed gripper arm 30, with a weakened section 44 of the tube providing a hinge. The flexible and extendable joint 34 can be extended by means of wires and/or rods 60 (or via an adjustment catheter 21, that also may push out the papillary anchor 9), which may apply a force to stretch elastic elements of the joint 34. This extension is used to move the papillary anchor 9, within its housing part 8, to place it against the papillary muscle 26, or close to it, since the wires/rods along with the papillary anchor 8 within the distal housing part 8 move with the housing 8 as the joint 34 extends. This can be due to friction between the papillary anchor 9 (or a papillary anchor push tube) and the internal surface of the distal part 8 of the housing section. The position can be confirmed by 3D ultrasound and/or other available sources.

When the distal end of the distal part 8 meets the body tissue, and as further force is applied the counterforce from the body tissue eventually surpasses the forces holding the papillary anchor 9 in place, at this point tissue is pushed flat below the base of the distal part 8 giving a maximal chance of placing all pins 62 of the papillary anchor 9 correctly in tissue, and force can be applied to the papillary anchor 9 so that the ends of the pins 62 then move beyond the distal end of the distal part 8 to meet the body tissue. This may be done via additional force on the papillary anchor 9 from rods or wires 60 or extending the adjustment catheter 21, or advantageously it may be done through a pre-tension on the papillary anchor 9 (or friction between the adjustment catheter 21 and the distal part 8) that is held by friction with the distal part until the forces from the body tissue on the distal part 8 changes the balance of forces with the friction sufficiently so that the papillary anchor 9 ejects in a way similar to a paper stapler. As the papillary anchor 9 is ejected the pins 62 fold out and form into the hook shape of the unconstrained papillary anchor 9 to thereby engage with the body tissue 26. At this point the connection can be pull tested by operator, and/or visually confirmed on x-ray and/or ultrasound. If the connection is not satisfactory, the papillary anchor 9 can be pulled back into the distal part 8 and re-placed to attempt an improved coupling of the anchor 9 with the body tissue 26.

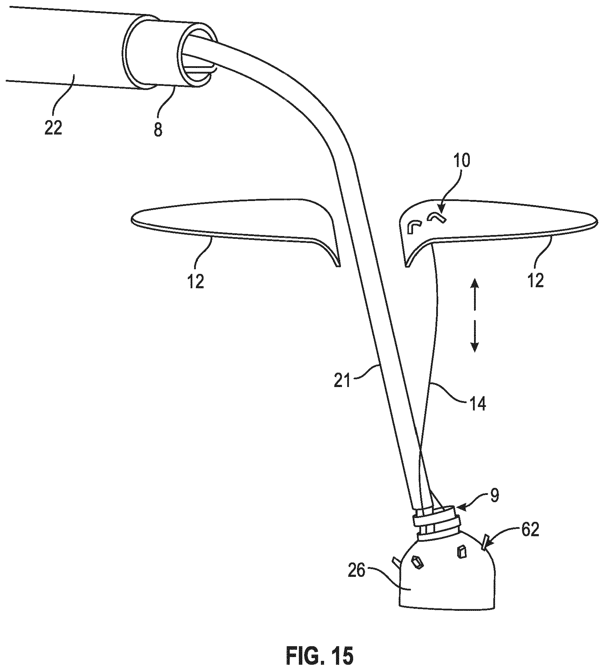

Figure 15 shows the possible next steps. The main part 4, 8 of the device is retracted to minimize influence on the moving leaflets 12. An adjustment catheter 21, which may comprise a Z-shaped fork 20 at its distal end as shown in Figures 20 to 22, can remain at the papillary anchor 9. The length of the artificial chordae line 14 can be adjusted with a wire from the outside. The length is continuously adjusted and the functioning of the leaflet 12 is monitored. The length of the artificial chordae line 14 can be reduced by pulling the chord wire back through the catheter. The length can also be increased by pushing the chord wire, which will slacken the artificial chordae line 14 and allow the movement of the leaflet 12 to pull it out of the adjustment catheter 21. The small size of the adjustment catheter 21 means that the effect of the device on the functioning of the leaflet 12 is minimised. The right length for the artificial chordae line 14 is confirmed with 3D ultrasound and/or other available sources.

When the correct length is confirmed then the device is disengaged from the papillary anchor 9. This process also locks the artificial chordae line 14 in place and cuts off any excess, which is retained in the catheter and withdrawn from the body when the catheter is removed. Figures 20 to 22 include more detail of the Z-shaped fork 20 and the cutting piece 18, as discussed below. The Z-shaped fork is used to hold open a locking segment 28 of the papillary anchor 9. The locking segment 28 is a band of the papillary anchor 9 that can be flexed to open a gap for the artificial chordae line 14 to pass through. In the natural shape of the papillary anchor 9, when no forced is applied, this locking segment 28 fits closely with the remainder of the anchor 9 and so it will hold the artificial chordae line 14 in place. The Z-shaped fork 20 is used to hold the locking segment 28 open until the artificial chordae line 14 is the correct length. The cutting piece 18 cuts the artificial chordae line 14, which is pulled against the blade when the adjustment process is completed.

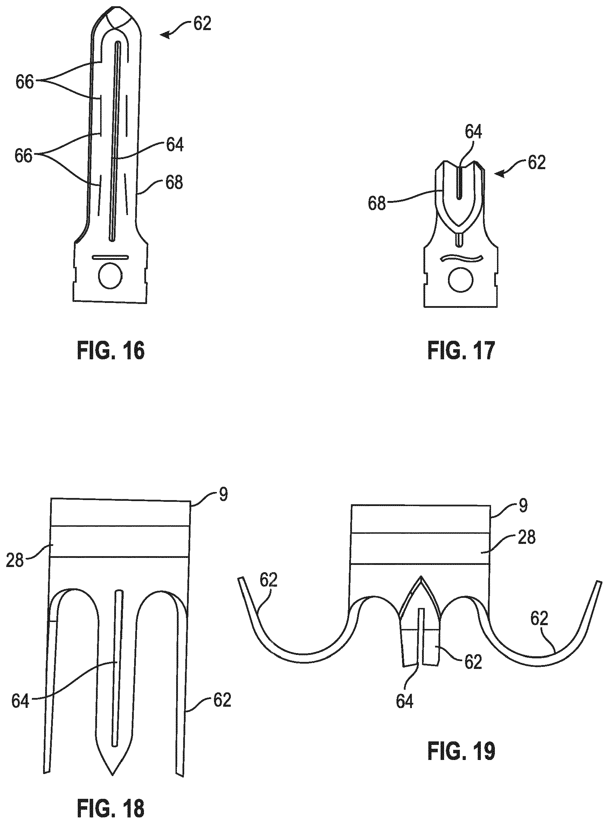

Figures 16 to 19 include more details of the papillary anchor 9, including its hooks 62 which are formed by curving pins 62. Figures 16 and 17 show one possible form for the hooks 62, with a central slit 64 and a series of holes 66 threaded with a suture 68. As discussed above, this suture 68 and the holes 66 can allow the hooks 62 to better engage with body tissue during healing, as well as keeping the material of the hooks 62 connected to the main body of the papillary anchor 9 in the event of a breakage. Figure 16 shows the folded/constrained shape of the hook 62, which is also the shape of a tine formed in a tubular section during manufacture of the anchor 9, prior to heat setting to form the curve. Figure 17 shows the curved form of the hook 62, i.e. the unfolded/unconstrained form.

Figures 18 and 19 show an example of an entire papillary anchor 9, again illustrating the folded (Figure 18) and unfolded (Figure 19) configurations. This papillary anchor 9 includes hooks 62 with an opening in the form of a slit 64, which gives various advantages as discussed above, including better engagement with the body during healing as well as increased surface area without loss of flexibility.

The device can include a safety wire 72 that acts to prevent the papillary anchor 9 from escaping into the body in the event that it is not correctly placed. Once the locking and cutting have been done, and the papillary anchor 9 is seen to be secured to the papillary muscle 26 and to the leaflet anchor 10 then the safety wire 72 is cut.

In order to deploy the leaflet anchor then a U-rod can be used. This U-rod 30 would be housed within the gripper arm 30 and partly within the main part of the catheter, with a free end of the U-shape being used to push the leaflet anchor 10 (and ejector unit 36, where present) along the leaflet anchor tube 38.The U-rod has a bendable section so the gripper can open and close, while the U-rod is inside. Advantageously, this bendable section can act as a sort of a spring, applying a restoring force to return the gripper arm 30 to the closed position. The U-rod is made of a material with the ability to deform elastically to a high degree in order to allow for the bending of the bendable section. Suitable materials include shape memory materials, for example shape memory metals such as nitinol. A shape memory metal also has the advantage that the U-rod can be made stiff, which makes the transfer of force with the U-rod more efficient. The U-rod may consist of a thin nitinol wire and tubes on the outside of the wire, to make the U section stiffer. Alternatively, the U-rod could be made of several types of materials to achieve the required properties.

As noted above, imaging techniques such as 3-D ultrasound or fluoroscopy can be used when guiding the device and to confirm the correct location of the leaflet 12 within the gripper device 6. To assist in this, the echogenic properties of the device may be improved by abrasive blasting, mechanical texture or a special coating, for example an echogenic polymer coating. The gripper device 6 can also be provided with a detection system to confirm the location of the leaflet 12 within the gripper 6. In a modified gripper (not shown) a fluid based sensor system is provided. This uses holes on the gripping surface of the gripper housing 4. The holes are connected through tubes to a fluid supply, such as contrast fluid from a syringe. When the gripper pinches the leaflet (or other tissue), the holes will be blocked by tissue preventing the flow of fluid. This can be used to determine if the leaflet is in the correct position to deploy the leaflet anchor. The device could be built with various numbers of holes, for example three or four, with the combination of open and closed holes being used to determine the position of the leaflet/tissue within the gripper 4. If four valves are placed in a square pattern, two closed and two open valves could represent the correct position of the leaflet. In one example, the sensor system consists of one-four fluid channels that can be located in the instrument wall, opposite of the gripper arm, alternatively in the gripper arm tip. The channels are connected to ports on the instruments handle where they can be injected with a contrast fluid, which can be visible on either echocardiography or fluoroscopy. An absence (or reduction) of visible fluid and/or the increased resistance to inject fluid in both channels tells the operator that the leaflet is correctly placed prior to leaflet anchor deployment.

In another example a pump with a monitoring circuit constantly pumps a small amount of water through the tubes of the sensor. The detection circuit can detect pressure rise or change in the volume going through each tube, the rise in pressure can indicate which tubes that are obstructed and to some degree says something about how thick the tissue in the leaflet actually is (thinner tissue tend to cause less pressure rise, relative to thicker tissue). The monitor device can for example be equipped with simple LEDs that go green if leaflet is properly gripped. This will give physicians further confirmation (in addition to Ultrasound) that they have captured the leaflet correctly, which ultimately results in higher procedure success rates. In a slightly different embodiment the pump can be programmed to slowly pump fluid in and out of the tubes, which does not require additional fluid if the procedure takes long time.

The device may include a suture/line management system, to prevent tangling. Sutures may be held inside slits or tubes, until everything is ready for them to be released, this will reduce the chance of entanglement. The suture slit in the papillary housing 8 may be equipped with a one way "suture valve" cut from the nitinol tube itself, it will prevent native chordaes from entering the chordae channel.

The artificial chordae line 14 can be attached to the anchor(s) in several ways. For example, wire through holes with knots, welds or glue. The artificial chordae line 14 can be made of Gore-Tex^{®} suture material, or a thin nitinol wire. This preferred embodiment uses Gore-Tex^{®} since it is easier to cut once the length has been adjusted. The artificial chordae line 14 has a diameter of approximately 0.1-0.6 mm. The leaflet anchor 10 is approximately 1-2 mm in diameter, and approximately 4-6 mm in length (when straight).

The leaflet anchor pins can be cut with several different profiles to achieve different strength, and/or faster healing. Since the leaflet anchor 10 is cut from tubing using laser cutting then different shapes are easy to produce. The pins of the anchor may for example have a straight edge (minimum friction) or a profile for increased friction, such as a smooth or sharp saw tooth, or a barbed profile. The anchor shape can vary based on the requirements of the procedure. Different anchor designs could be available for a surgeon to select based on their assessment of the patient.

As with the leaflet anchor pins, the papillary anchor pins can be cut with several different shapes to achieve different pull out strength and/or faster healing. The pins of the anchor may for example have a straight edge (minimum friction) or a profile for increased friction, such as a smooth or sharp saw tooth, or a barbed profile. The anchor shape can vary based on the requirements of the procedure. Different anchor designs could be available for a surgeon to select based on their assessment of the patient.

Figures 20 to 22 illustrate interaction of the papillary anchor 9 with the chord and a cutting piece 18 of the catheter device. The cutting piece 18, is made of a suitable biocompatible material, preferably cut with laser and sharpened by grinding away some material. The material may for example be stainless steel, titanium or titanium alloy. Nitinol could also be used. The Z-shaped fork 20 is used to hold the locking segment 28 open to make room for the chord between the locking rings and locking segment 28 in the papillary anchor 9.

Once the papillary anchor 9 is placed and the delivery device is retracted, as discussed above, then a chordae-wire 14 is used to adjust the chordae length. An optional wire lock (not shown) can be pulled to gently pinch the artificial chordae line 14 in the temporary adjusted state during analysing of the length, the wire-chordae will in addition be held from the outside. Once the correct length is achieved, a locking wire 70 is pulled, which bends/retracts the Nitinol Z shape 20 and locks the chordae in place by releasing the locking segment 28. Then the cutting piece 18 is pulled and its nitinol knife engages with the artificial chordae line 14 as well as one strand of a papillary anchor holder suture 72. The papillary anchor 9 is now free from the adjustment and cutting device 18, 20.

The use of the Z shaped nitinol fork 20 to hold the locking segment 28 open allows the suture/chordae pathway to get a very gentle curve. It also allows the suture to come out of the device in line with the gripper opening. This is important to get as good as possible load conditions on the papillary anchor (Chorda comes out of the anchor in the correct place for optimal holding strength).

In one embodiment the cutter 18 is made from a thin sheet nitinol, which allows the blade to be pulled around a curved surface, to allow a minimal footprint of a relative long sliding action component (it can be pulled for example perpendicular to the cutting surface, taking up much less space). The Z-fork 20 can be produced from a laser cut heat set Nitinol sheet part, where certain sections can be grinded thinner, to obtain different thickness and flex along the part. It is possible to add in a simple temporary wire lock, when pulled it will gently squeeze the chordae 14 in order to maintain its temporary adjusted length, in addition to hold the wire that is connected to the chordae 14 on the outside (not in illustrations). Note that the supports inside the adjustment device 21 are not shown. The chamfer on the top part of adjustment "box" will allow the device to find the anchor 9 if it needs to be retrieved.

In one embodiment a push out tube connected to the papillary anchor 9 contains several markers that can be used as a rough reference point on the distance between the papillary anchor and the leaflet anchor, this could allow the physician to roughly adjust the chordae prior to do the final adjustments as they normally have a hunch about how long the final chordae length should be.

To prevent the cutter 18 from exceeding its desired range of motion, the cutter 18 may be equipped with two stopping features disposed at an upper and lower end of the cutter 18. To prevent the cutter 18 from moving further than its upper position in the housing, a cutter wire may be threaded through the housing and/or the cutter to stop the cutter 18 in an upper position. Even if the cutter wire were to break, the cutter 18 and a wire attached to the cutter operating it cannot escape from an upwards end of the housing as both are contained within the housing. To prevent the cutter 18 from moving further than its lower position in the housing, a cam may be used.

The shaft of the part of the catheter device 2 which houses the cutter 18 and the adjustment device 21 (not shown) can be constructed with two lumens: one chordae lumen and one cutter lumen. The construction can be reinforced with braiding around the chordae lumen (the shaft may also include any lumens required to house pullwires used for operating the device, which may also be reinforced with braiding). In addition to the braiding, a wire made out of Kevlar or another similar material may be implemented in the construction running along the length of the shaft, to increase the tensile strength of the device 2. Additionally or alternatively, a composite tube may be positioned around the lumens. The components and tubing of the shaft can also be embedded in a soft polymer, such as Pebax (e.g. by Pebax reflow), to allow for sufficient flex. The composite tubing may also be anchored in the distal end to prevent the tubing from being torn out of the soft polymer during actuation of the cutter wire. The composite tubing may be anchored in the distal end with, for example, a flat ribbon coil, a stainless steel hypotube ring, or a stainless steel collar.

The braid around the chordae lumen may comprise a laser cut hypotube, which increases the tensile and compression strength of the of the shaft construction. The laser cut hypotube can be 'flex tailored' such that different sections have different flex patterns to accommodate a desired movement of the shaft. The laser cut hypotube can also be welded directly onto the head of the cutter 18. The strong bond between the cutter head and the laser cut hypotube allows for more reliable retrieval of the papillary anchor if readjustment is desired. A braided composite tubing may be disposed outside the laser cut hypotube to form the wire lumens.

In some cases the natural chordae could be a problem for the device. There is a risk of fouling if one of the existing chordae is caught in the hole provided for the exit of the new artificial chordae line 14. One way to eliminate this is to have a one-way chord exit so that the artificial chordae line 14 can only go out of the device, and not in, although this feature is not essential.

Inside the papillary housing 8 there may be small notches in the walls to hold the pins of the papillary anchor 9 and prevent the papillary anchor 9 from rotating so that the pins could fold out in the opening for the new chord 14.

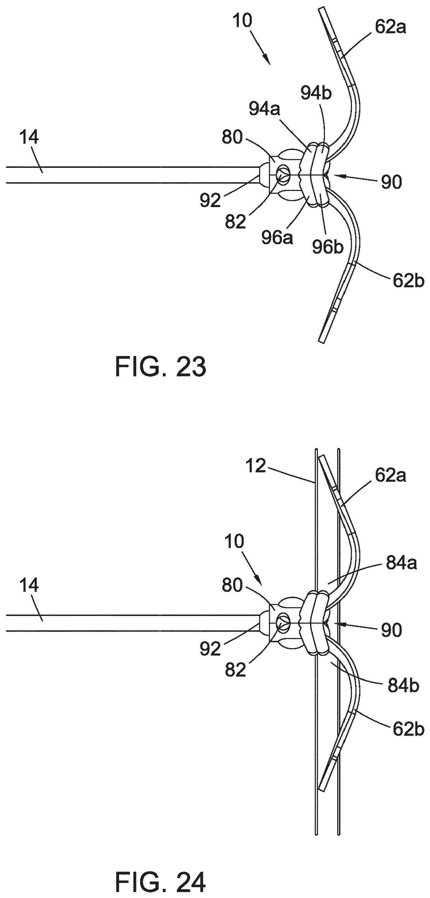

Figure 23 shows a leaflet anchor 10 in combination with a line 14. The leaflet anchor 10 comprises a number of hooks 62 extending from an anchor body 80 of the leaflet anchor 10. The hooks 62 are in the unfolded position. The hooks 62 extend from a base of the anchor body 80. The anchor body 80 comprises a number of threading holes 82 accommodating at least part of the knotting configuration 90. Whilst a single threading hole 82 is visible in the Figures, it will be appreciated that multiple threading holes can be located around a circumference of the anchor body 80. The line 14 is seen passing through two threading holes 82, for example, in the plane of the page of Figure 23.

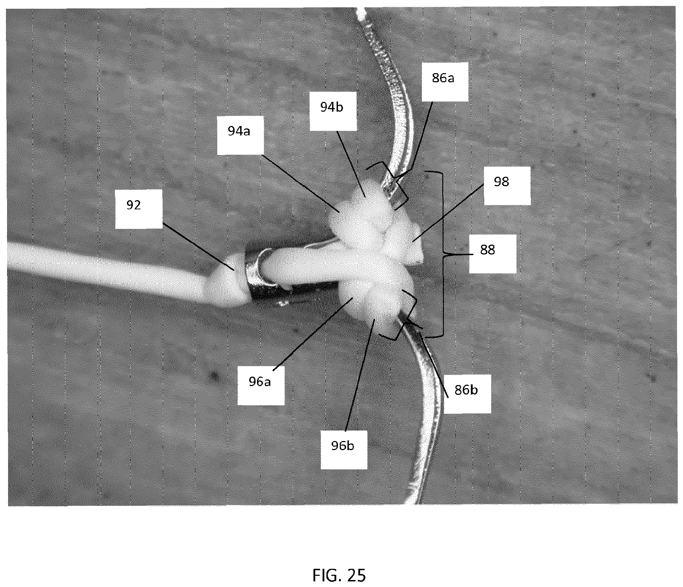

The line 14 is joined to the leaflet anchor 10 is joined to the line 14 by a knotting configuration 90. The knotting configuration 90 comprises a plurality of loops and a plurality of knots. A first knot 92 is located at a proximal end of the anchor body 80, located within the anchor body 80. The line 14 is then looped twice around the first hook 62a by a first loop 94a and a second loop 94b, and is then looped twice around the second hook 62b by a third loop 96a and a fourth loop 96b. Whilst the line 14 shown is looped twice around each hook 62a, 62b in Figures 23 to 25, the line 14 may only be looped once around each hook 62a, 62b. Equally, the line 14 may be looped more than twice around each hook 62a, 62b. Shown in Figure 25, a second knot 98 is located at a distal end of the anchor 14. The second knot 98 is adjacent to the second loop 94b and the fourth loop 96b. The second knot 98 comprises the fourth loop 96b.

Whilst not shown in any of Figures 23 to 25, the end of the line 14 distal to the leaflet anchor 10 can be attached to the papillary anchor 9. The line 14 can be joined to the papillary anchor 9 by the locking segment 28. The leaflet anchor 10 in combination with the line 14 is suitable for use as the leaflet anchor 10 in any of the devices and/or methods discussed above.

Figure 24 shows the leaflet anchor 10 in combination with the line 14 implanted in body tissue 12, which in this case the mitral leaflet 12. The hooks 62 engage the mitral leaflet 12 as described above. As the hooks 62 pierce the mitral leaflet 12, entry sites 84 form around each hook 62. These entry sites 84 each or collectively may be regarded as an entry site 84 of the anchor 10 when implanted in the mitral leaflet 12. The entry sites 84 may generally comprise narrow channels and/or passageways around the length of the hooks 62 through the mitral leaflet 12.

The leaflet anchor 10 is implanted in the mitral leaflet 12 from the ventricular side, and thus the side of the mitral leaflet 12 that the anchor body 80 of the leaflet anchor 10 is located may be regarded as a high pressure side of the leaflet 12. During the cardiac cycle, blood may be forced through the entry sites 84 due to the fluid pressure generated by contraction of the heart. The blood forced through the entry sites 84 may produce a high-velocity jet into the atrial side of the leaflet 12, i.e. a low pressure side. Flow of blood through the leaflet via the entry sites 84 will inhibit tissue regrowth where the hooks pass through the tissue of the leaflet. To reach the entry sites 84 the blood may flow at high pressure and/or velocity between the anchor body 80 of the leaflet anchor 10 and the mitral leaflet 12, which exerts a force on the anchor 10 at the site of implantation in the mitral leaflet 12. This force may act to drive the anchor body 80 of the leaflet anchor 10 away from the mitral leaflet 12. The exerted force can therefore weaken the tensile strength of the anchor 14 when implanted in the mitral leaflet 12. For example, the presence of high pressure flow between the anchor body 80 and the mitral leaflet 12 may discourage the ingrowth of tissue around the base of the anchor 10 which could otherwise strengthen the implantation of the anchor 10.

The provision of the knotting configuration 90 provides a sealing effect against the entry site 84, which prevents and/or impedes the flow of blood through them. This helps to prevent the flow of high-velocity jets through the entry site 84 which could otherwise prevent the ingrowth of tissue around the anchor 10. The loops 94b, 96b each respectively provide a first sealing surface 86a, 86b which seal the entry site of the hook 62a, 62b the loop 94b, 96b is wrapped around. The first sealing surfaces 86a, 86b provide a localised sealing effect around each entry site 84a, 84b created by the respective hook 62a, 62b it is wrapped around.

The knotting configuration 90 also provides a second sealing surface 88. In contrast to the first sealing surfaces 86a, 86b, the second sealing surface 88 provides an overall sealing effect which captures all the entry sites 84 created by all the hooks 62. The second sealing surface 88 is generally formed by the second knot 98, the second loop 94b and the fourth loop 96b. As the second knot 98, the second loop 94b and the fourth loop 96b are adjacent one another, they form a 'psuedo-loop' due to their contact with one another, which thus in turn provides the overall sealing effect.

In combination, the first sealing surfaces 86a, 86b and the second sealing surface 88 provide a plugging effect which prevents and/or impedes the flow of high-velocity jets through the entry sites 84 of the anchor 10 when implanted in the mitral leaflet 12. This may encourage the ingrowth of body tissue around the base of the anchor 10, which overall improves the tensile strength of the anchor.

The plugging effect may be realised due to the pressure differential of the highpressure side and the low-pressure side across the entry sites 84. The sealing surfaces 86, 88 may be suctioned to the mitral leaflet 12 around the entry sites 84. Additionally, the anchor body 80 can act to compress the loops 94, 96 to the mitral leaflet 12 to further strengthen the sealing surfaces 86, 88. The anchor body 80 can compress the sealing surfaces 86, 88 due to a fluid pressure acting on the anchor body 80 to compress the anchor body 80, and hence the sealing surfaces 86, 88 against the mitral leaflet 12; and due to a springback effect owing to the elastic properties of the hooks 62, which act to 'pull' the leaflet anchor 10 into the mitral leaflet 12 during implantation.

The knotting configuration 90 also provides an increased surface area for body tissue to grow around the base of the anchor 10. To encourage the growth of body tissue around the knotting configuration 90 and thus the base of the anchor 10, the line 14, or at least an outer coating of the line 14, may be formed of a biocompatible material, such as ePTFE.

The knotting configuration 90 can be regarded as a plugging device as, in accordance with the above description, the knotting configuration 90 and particularly the sealing surfaces 86, 88 of the knotting device plug the entry sites 84 of the anchor 10 in body tissue such as the mitral valve.

## Claims

1. An anchor configuration for implantation in body tissue (12), the anchor configuration comprising:
an anchor (10) comprising a number of hooks (62a, 62b) for engagement with the body tissue (12) and having a folded and unfolded position, wherein the anchor (10) is made of an elastic material such that it can be elastically deformed into the folded position by application of a constraining force, and will return to the unfolded position when no constraining force is applied; and
a plugging device (90) for enhancing contact with the body tissue (12);
wherein the plugging device (90) is for combining with one or more parts of the anchor (10) to provide the enhanced contact;
wherein at least one of the hooks (62a, 62b) is encircled by the plugging device (90);
wherein the plugging device (90) is a line (14), the line (14) in combination with the anchor (10);
said anchor configuration being **characterized in that**
wherein the line (14) is joined to the anchor by a knotting configuration (90) comprising a plurality of loops (94a, 94b, 96a, 96b) around the anchor (10);
wherein the at least one hook (62a, 62b) is encircled by at least one loop of the plurality of loops (94a, 94b, 96a, 96b); and
wherein the line (14) is configured to seal an entry site (84a, 84b) between the hooks (62a, 62b) and the body tissue (12) when the anchor (10) is implanted in the body tissue (12).

2. An anchor configuration as claimed in claim 1, wherein the anchor (10) comprises an anchor body (80), wherein the hooks (62a, 62b) extend from a base of the anchor body (80).

3. An anchor configuration as claimed in claim 2, wherein the anchor body (80) is configured to compress the line (14) against the body tissue (12) when the hooks (62a, 62b) are in the unfolded position and the anchor (10) is implanted in the body tissue (12).

4. An anchor configuration as claimed in claim 2 or 3, wherein the anchor body (80) is a tubular body, and wherein at least part of the knotting configuration (90) is located within the tubular body.

5. An anchor configuration as claimed in claim 2, 3 or 4, wherein the anchor body (80) comprises at least two threading holes (82), wherein the threading holes (82) accommodate at least part of the knotting configuration (90).

6. An anchor configuration as claimed in any preceding claim, wherein a portion of the line (14) encircling the at least one hook (62a, 62b) is configured to provide a first sealing surface (85a, 85b; 86a; 86b) around a respective entry site (84a, 84b) of the at least one hook (62a, 62b), and wherein the plugging device (89; 90) is configured to provide a second sealing surface (87; 88) capturing all of the entry sites (84a; 84b) of all of the hooks (62a; 62b).

7. An anchor configuration as claimed in any preceding claim, wherein the plugging device (90) is formed of a biocompatible material, optionally wherein the biocompatible material is ePTFE.

8. An anchor configuration as claimed in any preceding claim, wherein the knotting configuration (90) comprises at least two knots.

9. A catheter device (2) comprising an anchor deployment mechanism and an anchor configuration as claimed in any preceding claim.

10. A catheter device (2) as claimed in claim 9, wherein the anchor (10) is a leaflet anchor for implantation into a leaflet (12) of the heart, and wherein the anchor deployment mechanism is hence a leaflet anchor deployment mechanism.

11. A catheter device (2) as claimed in claim 9 or 10, wherein the line (14) is an artificial chordae line and wherein the catheter device (2) is for implanting the anchor (10) during a procedure for implanting the artificial chordae line into the heart, the catheter device (2) comprising: the anchor (10), the anchor deployment mechanism for deploying the anchor, and an ejector unit (36) for releasably grasping the anchor (10);
optionally comprising a rod (30) for deployment of the anchor (10), wherein the rod (30) is a U-rod in order to allow for a pushing force directed toward the proximal end of the catheter device (2).

12. A catheter device (2) as claimed in claim 11, wherein the anchor deployment mechanism allows for retraction and repositioning of the anchor (10) after deployment of the anchor (10) into the body tissue (12) via the ejector unit (36), wherein the ejector unit (36) has a grasping device (50) with a first configuration arranged to permit deployment of the anchor (10) into the body tissue (12) without disengagement of the anchor (10) from the ejector unit (36), and a second configuration in which the anchor (10) is reversibly released from the ejector unit (36); wherein in the first configuration the grasping device (50) of the ejector unit (36) grasps the centre of the anchor (10), whilst the hooks (62a, 62b) of the anchor (10) are unimpeded by the grasping device (50) to enable it to be implanted in the body tissue (10); and wherein in the second configuration the grasping device (50) of the ejector unit (36) is disengaged from the anchor (10).

13. A catheter device (2) as claimed in claim 12, wherein the anchor (10) comprises tabs or recesses either side of the width of the anchor (10) at its centre in order to allow for the grasping device (50) of the ejector unit (36) to engage with the anchor (10); and/or
wherein prior to deployment the ejector unit (36) is placed within the anchor deployment mechanism inboard of the anchor (10) and when the ejector unit (36) and anchor (10) are within the anchor deployment mechanism the ejector unit (36) holds the anchor (10) with the grasping device (50) in the first configuration.

14. A catheter device as claimed in claim 12 or 13, wherein the anchor (10) is a leaflet anchor (10) for implantation into a leaflet (12) of the heart, and wherein the anchor deployment mechanism is hence a leaflet anchor deployment mechanism, and wherein the leaflet anchor (10) and the ejector unit (36) are housed inside an anchor tube (38) of the leaflet anchor deployment mechanism prior to deployment, with the ejector unit (36) further inside the anchor tube (38) than the anchor (10);
wherein the grasping device (50) optionally comprises two or more grappling hooks arranged to engage with the anchor (10) at their ends when in the first configuration, and wherein the grasping device (50) is arranged to engage and disengage from the anchor (10) via a radial movement of the grappling hooks relative to the anchor tube (38).

15. A method of manufacture of the anchor configuration as claimed in any of claims 1 to 8, the method comprising: forming the anchor (10) from an elastic material; and joining the line (14) to the anchor (10).

## Patentansprüche

1. Ankerkonfiguration zur Implantation in Körpergewebe (12), wobei die Ankerkonfiguration umfasst:
einen Anker (10), der eine Anzahl von Haken (62a, 62b) zum Eingriff mit dem Körpergewebe (12) umfasst und eine gefaltete und eine ungefaltete Position aufweist, wobei der Anker (10) aus einem elastischen Material hergestellt ist, so dass er durch Aufbringen einer Begrenzungskraft elastisch in die gefaltete Position verformt werden kann und in die ungefaltete Position zurückkehren wird, wenn keine Begrenzungskraft aufgebracht wird; und
eine Verschlussvorrichtung (90) zur Verbesserung eines Kontakts mit dem Körpergewebe (12);
wobei die Verschlussvorrichtung (90) dazu dient, mit einem oder mehreren Teilen des Ankers (10) kombiniert zu werden, um den verbesserten Kontakt bereitzustellen;
wobei mindestens einer der Haken (62a, 62b) von der Verschlussvorrichtung (90) umgeben ist;
wobei die Verschlussvorrichtung (90) ein Strang (14) ist, der Strang (14) in Kombination mit dem Anker (10);
wobei die Ankerkonfiguration **dadurch gekennzeichnet ist, dass**
wobei der Strang (14) mit dem Anker durch eine Knotenkonfiguration (90) verbunden ist, die eine Vielzahl von Schlaufen (94a, 94b, 96a, 96b) um den Anker (10) herum umfasst;
wobei der mindestens eine Haken (62a, 62b) von mindestens einer Schlaufe der Vielzahl von Schlaufen (94a, 94b, 96a, 96b) umgeben ist; und
wobei der Strang (14) so konfiguriert ist, dass er eine Eintrittsstelle (84a, 84b) zwischen den Haken (62a, 62b) und dem Körpergewebe (12) abdichtet, wenn der Anker (10) in das Körpergewebe (12) implantiert ist.

2. Ankerkonfiguration nach Anspruch 1, wobei der Anker (10) einen Ankerkörper (80) umfasst, wobei sich die Haken (62a, 62b) von einer Basis des Ankerkörpers (80) erstrecken.

3. Ankerkonfiguration nach Anspruch 2, wobei der Ankerkörper (80) so konfiguriert ist, dass er den Strang (14) gegen das Körpergewebe (12) drückt, wenn sich die Haken (62a, 62b) in der ungefalteten Position befinden und der Anker (10) in das Körpergewebe (12) implantiert ist.

4. Ankerkonfiguration nach Anspruch 2 oder 3, wobei der Ankerkörper (80) ein röhrenförmiger Körper ist und wobei sich mindestens ein Teil der Knotenkonfiguration (90) innerhalb des röhrenförmigen Körpers befindet.

5. Ankerkonfiguration nach Anspruch 2, 3 oder 4, wobei der Ankerkörper (80) mindestens zwei Gewindebohrungen (82) umfasst, wobei die Gewindebohrungen (82) mindestens einen Teil der Knotenkonfiguration (90) aufnehmen.

6. Ankerkonfiguration nach einem vorstehenden Anspruch, wobei ein Abschnitt des Strangs (14), der den mindestens einen Haken (62a, 62b) umgibt, so konfiguriert ist, dass er eine erste Dichtfläche (85a, 85b; 86a; 86b) um eine jeweilige Eintrittsstelle (84a, 84b) des mindestens einen Hakens (62a, 62b) bereitstellt, und wobei die Verschlussvorrichtung (89; 90) so konfiguriert ist, dass sie eine zweite Dichtfläche (87; 88) bereitstellt, die alle Eintrittsstellen (84a; 84b) aller Haken (62a; 62b) erfasst.

7. Ankerkonfiguration nach einem vorstehenden Anspruch, wobei die Verschlussvorrichtung (90) aus einem biokompatiblen Material gebildet ist, optional wobei das biokompatible Material optional ePTFE ist.

8. Ankerkonfiguration nach einem vorstehenden Anspruch, wobei die Knotenkonfiguration (90) mindestens zwei Knoten umfasst.

9. Kathetervorrichtung (2), umfassend einen Anker-Entfaltungsmechanismus und eine Ankerkonfiguration nach einem vorstehenden Anspruch.

10. Kathetervorrichtung (2) nach Anspruch 9, wobei der Anker (10) ein Klappenanker zur Implantation in eine Klappe (12) eines Herzens ist und wobei der Anker-Entfaltungsmechanismus somit ein Klappenanker-Entfaltungsmechanismus ist.

11. Kathetervorrichtung (2) nach Anspruch 9 oder 10, wobei der Strang (14) ein künstlicher Chordae-Strang ist und wobei die Kathetervorrichtung (2) zum Implantieren des Ankers (10) während eines Verfahrens zum Implantieren des künstlichen Chordae-Strangs in das Herz dient, wobei die Kathetervorrichtung (2) umfasst: den Anker (10), den Anker-Entfaltungsmechanismus zum Entfalten des Ankers und eine Auswerfereinheit (36) zum lösbaren Greifen des Ankers (10);
optional umfassend einen Stab (30) zum Entfalten des Ankers (10), wobei der Stab (30) ein U-Stab ist, um eine zu dem proximalen Ende der Kathetervorrichtung (2) gerichtete Schubkraft zu ermöglichen.

12. Kathetervorrichtung (2) nach Anspruch 11, wobei der Anker-Entfaltungsmechanismus ein Zurückziehen und Neupositionieren des Ankers (10) nach einem Entfalten des Ankers (10) in das Körpergewebe (12) über die Auswerfereinheit (36) ermöglicht, wobei die Auswerfereinheit (36) eine Greifvorrichtung (50) mit einer ersten Konfiguration aufweist, die so angeordnet ist, dass sie das Entfalten des Ankers (10) in das Körpergewebe (12) ohne Lösen des Ankers (10) von der Auswerfereinheit (36) ermöglicht, und einer zweiten Konfiguration, in der der Anker (10) reversibel von der Auswerfereinheit (36) gelöst wird; wobei in der ersten Konfiguration die Greifvorrichtung (50) der Auswerfereinheit (36) die Mitte des Ankers (10) greift, während die Haken (62a, 62b) des Ankers (10) von der Greifvorrichtung (50) nicht behindert werden, damit er in das Körpergewebe (10) implantiert werden kann; und wobei in der zweiten Konfiguration die Greifvorrichtung (50) der Auswerfereinheit (36) von dem Anker (10) gelöst ist.

13. Kathetervorrichtung (2) nach Anspruch 12, wobei der Anker (10) beiderseits der Breite des Ankers (10) in dessen Mitte Laschen oder Aussparungen umfasst, um zu ermöglichen, dass die Greifvorrichtung (50) der Auswerfereinheit (36) mit dem Anker (10) in Eingriff kommen kann; und/oder
wobei vor dem Entfalten die Auswerfereinheit (36) innerhalb des Anker-Entfaltungsmechanismus innerhalb des Ankers (10) platziert ist und wenn sich die Auswerfereinheit (36) und der Anker (10) innerhalb des Anker-Entfaltungsmechanismus befinden, die Auswerfereinheit (36) den Anker (10) mit der Greifvorrichtung (50) in der ersten Konfiguration hält.

14. Kathetervorrichtung nach Anspruch 12 oder 13, wobei der Anker (10) ein Klappenanker (10) zur Implantation in eine Klappe (12) eines Herzens ist, und wobei der Anker-Entfaltungsmechanismus somit ein Klappenanker-Entfaltungsmechanismus ist, und wobei der Klappenanker (10) und die Auswerfereinheit (36) vor dem Entfalten in einem Ankerrohr (38) des Klappenanker-Entfaltungsmechanismus untergebracht sind, wobei die Auswerfereinheit (36) weiter innerhalb des Ankerrohrs (38) angeordnet ist als der Anker (10);
wobei die Greifvorrichtung (50) optional zwei oder mehr Greifhaken umfasst, die so angeordnet sind, dass sie in der ersten Konfiguration mit ihren Enden in den Anker (10) eingreifen, und wobei die Greifvorrichtung (50) so angeordnet ist, dass sie durch eine radiale Bewegung der Greifhaken relativ zu dem Ankerrohr (38) in den Anker (10) eingreift und sich aus diesem löst.

15. Verfahren zur Herstellung der Ankerkonfiguration nach einem der Ansprüche 1 bis 8, wobei das Verfahren umfasst: Bilden des Ankers (10) aus einem elastischen Material; und Verbinden des Strangs (14) mit dem Anker (10).

## Revendications

1. Configuration d'ancre pour l'implantation dans un tissu corporel (12), la configuration d'ancre comprenant :
une ancre (10) comprenant un certain nombre de crochets (62a, 62b) destinés à entrer en contact avec le tissu corporel (12) et présentant une position pliée et dépliée, dans laquelle l'ancre (10) est constituée d'un matériau élastique de telle sorte qu'elle puisse être déformée élastiquement dans la position pliée par l'application d'une force contraignante, et qu'elle revienne dans la position dépliée lorsqu'aucune force contraignante n'est appliquée ; et
un dispositif d'obturation (90) pour améliorer le contact avec le tissu corporel (12) ;
dans lequel le dispositif d'obturation (90) est destiné à être combiné avec une ou plusieurs parties de l'ancre (10) afin d'améliorer le contact ;
dans lequel au moins l'un des crochets (62a, 62b) est entouré par le dispositif d'obturation (90) ; dans lequel le dispositif d'obturation (90) est une ligne (14), la ligne (14) étant combinée avec l'ancre (10) ;
ladite configuration d'ancre étant **caractérisée en ce que**
dans lequel la ligne (14) est reliée à l'ancre par une configuration de nouage (90) comprenant une pluralité de boucles (94a, 94b, 96a, 96b) autour de l'ancre (10) ;
dans lequel l'au moins un crochet (62a, 62b) est entouré par au moins une boucle de la pluralité de boucles (94a, 94b, 96a, 96b) ; et
dans lequel la ligne (14) est configurée pour fermer hermétiquement un site d'entrée (84a, 84b) entre les crochets (62a, 62b) et le tissu corporel (12) lorsque l'ancre (10) est implantée dans le tissu corporel (12).

2. Configuration d'ancre selon la revendication 1, dans laquelle l'ancre (10) comprend un corps d'ancre (80), dans laquelle les crochets (62a, 62b) s'étendent à partir d'une base du corps d'ancre (80).

3. Configuration d'ancre selon la revendication 2, dans laquelle le corps d'ancre (80) est configuré pour comprimer la ligne (14) contre le tissu corporel (12) lorsque les crochets (62a, 62b) sont dans la position dépliée et que l'ancre (10) est implantée dans le tissu corporel (12).

4. Configuration d'ancre selon la revendication 2 ou 3, dans laquelle le corps d'ancre (80) est un corps tubulaire, et dans laquelle au moins une partie de la configuration de nouage (90) est située à l'intérieur du corps tubulaire.

5. Configuration d'ancre selon la revendication 2, 3 ou 4, dans laquelle le corps d'ancre (80) comprend au moins deux trous d'enfilage (82), dans laquelle les trous d'enfilage (82) accueillent au moins une partie de la configuration de nouage (90).

6. Configuration d'ancre selon la revendication selon une quelconque revendication précédente, dans laquelle une partie de la ligne (14) entourant l'au moins un crochet (62a, 62b) est configurée pour fournir une première surface d'étanchéité (85a, 85b ; 86a ; 86b) autour d'un site d'entrée (84a, 84b) respectif de l'au moins un crochet (62a, 62b), et dans laquelle le dispositif d'obturation (89 ; 90) est configuré pour fournir une seconde surface d'étanchéité (87 ; 88) capturant tous les sites d'entrée (84a ; 84b) de tous les crochets (62a ; 62b).

7. Configuration d'ancre selon la revendication selon une quelconque revendication précédente, dans laquelle le dispositif d'obturation (90) est constitué d'un matériau biocompatible, facultativement dans laquelle le matériau biocompatible est du PTFEe.

8. Configuration d'ancre selon la revendication selon une quelconque revendication précédente, dans laquelle la configuration de nouage (90) comprend au moins deux nœuds.

9. Dispositif de cathéter (2) comprenant un mécanisme de déploiement d'ancre et une configuration d'ancre selon une quelconque revendication précédente.

10. Dispositif de cathéter (2) selon la revendication 9, dans lequel l'ancre (10) est une ancre de valvule destinée à être implantée dans une valvule (12) du cœur, et dans lequel le mécanisme de déploiement d'ancre est donc un mécanisme de déploiement d'ancre de valvule.

11. Dispositif de cathéter (2) selon la revendication 9 ou 10, dans lequel la ligne (14) est une ligne de cordage artificielle et dans lequel le dispositif de cathéter (2) sert à implanter l'ancre (10) au cours d'une procédure d'implantation de la ligne de cordage artificielle dans le cœur, le dispositif de cathéter (2) comprenant : l'ancre (10), le mécanisme de déploiement d'ancre pour déployer l'ancre, et une unité d'éjection (36) pour saisir de manière libérable l'ancre (10) ;
comprenant facultativement une tige (30) pour le déploiement de l'ancre (10), dans lequel la tige (30) est une tige en forme de U afin de permettre une force de poussée dirigée vers l'extrémité proximale du dispositif de cathéter (2).

12. Dispositif de cathéter (2) selon la revendication 11, dans lequel le mécanisme de déploiement d'ancre permet la rétraction et le repositionnement de l'ancre (10) après le déploiement de l'ancre (10) dans le tissu corporel (12) par l'intermédiaire de l'unité d'éjection (36), dans lequel l'unité d'éjection (36) présente un dispositif de préhension (50) avec une première configuration conçue pour permettre le déploiement de l'ancre (10) dans le tissu corporel (12) sans séparation de l'ancre (10) de l'unité d'éjection (36), et une seconde configuration dans laquelle l'ancre (10) est libérée de manière réversible de l'unité d'éjection (36) ; dans lequel dans la première configuration, le dispositif de préhension (50) de l'unité d'éjection (36) saisit le centre de l'ancre (10), tandis que les crochets (62a, 62b) de l'ancre (10) ne sont pas entravés par le dispositif de préhension (50) pour permettre son implantation dans le tissu corporel (10) ; et dans lequel, dans la seconde configuration, le dispositif de préhension (50) de l'unité d'éjection (36) est séparé de l'ancre (10).

13. Dispositif de cathéter (2) selon la revendication 12, dans lequel l'ancre (10) comprend des languettes ou des évidements de part et d'autre de la largeur de l'ancre (10) en son centre afin de permettre au dispositif de préhension (50) de l'unité d'éjection (36) d'entrer en contact avec l'ancre (10) ; et/ou
dans lequel, avant le déploiement, l'unité d'éjection (36) est placée dans le mécanisme de déploiement d'ancre à l'intérieur de l'ancre (10) et, lorsque l'unité d'éjection (36) et l'ancre (10) sont dans le mécanisme de déploiement d'ancre, l'unité d'éjection (36) maintient l'ancre (10) avec le dispositif de préhension (50) dans la première configuration.

14. Dispositif de cathéter selon la revendication 12 ou 13, dans lequel l'ancre (10) est une ancre de valvule (10) destinée à être implantée dans une valvule (12) du cœur, et dans lequel le mécanisme de déploiement d'ancre est donc un mécanisme de déploiement d'ancre de valvule, et dans lequel l'ancre de valvule (10) et l'unité d'éjection (36) sont logées à l'intérieur d'un tube d'ancre (38) du mécanisme de déploiement d'ancre de valvule avant le déploiement, l'unité d'éjection (36) se trouvant plus loin à l'intérieur du tube d'ancre (38) que l'ancre (10) ;
dans lequel le dispositif de préhension (50) comprend facultativement deux crochets de préhension ou plus disposés pour entrer en contact avec l'ancre (10) au niveau de leurs extrémités lorsqu'ils sont dans la première configuration, et dans lequel le dispositif de préhension (50) est conçu pour entrer en contact avec l'ancre (10) et se séparer de celle-ci au moyen d'un mouvement radial des crochets de préhension par rapport au tube d'ancre (38).

15. Procédé de fabrication de la configuration d'ancre selon l'une quelconque des revendications 1 à 8, le procédé comprenant : la formation de l'ancre (10) à partir d'un matériau élastique ; et la liaison de la ligne (14) à l'ancre (10).
